# EUROPÄISCHE PATENTSCHRIFT

(11) **EP 2 714 773 B1**
(45) Veröffentlichungstag und Bekanntmachung des Hinweises auf die Patenterteilung: **22.08.2018**
(21) Anmeldenummer: 12727334.0
(22) Anmeldetag: 29.05.2012
(51) Int. Cl.: C08G 77/28, C08G 77/392, C09J 183/08

(54) **KIESELSÄURE(HETERO)POLYCOKONDENSATE MIT ORGANISCH POLYMERISIERBAREN GRUPPEN UND ENTWEDER SULFONAT- ODER SULFATGRUPPEN, DARAUS HERGESTELLTE ORGANISCHE POLYMERISATE SOWIE VERFAHREN ZUM HERSTELLEN DER POLYKONDENSATE**
SILICIC ACID (HETERO) POLYCONDENSATES COMPRISING ORGANICALLY POLYMERISABLE GROUPS AND EITHER SULPHONATE GROUPS OR SULPHATE GROUPS, ORGANIC POLYMERISATES PRODUCED THEREFROM, AND A METHOD FOR PRODUCING SAID POLYCONDENSATES
POLYCONDENSATS D'ACIDE (HÉTÉRO)SILICIQUE RENFERMANT DES GROUPES ORGANIQUEMENT POLYMÉRISABLES ET, SOIT DES GROUPES SULFONATE, SOIT DES GROUPES SULFATE, POLYMÉRISATS ORGANIQUES PRODUITS À PARTIR DE CES POLYCONDENSATS, ET PROCÉDÉ DE PRODUCTION DESDITS POLYCONDENSATS

(30) Priorität: 27.05.2011 DE 102011050672; 27.05.2011 EP 11167853
(43) Veröffentlichungstag der Anmeldung: 09.04.2014
(73) Patentinhaber: Fraunhofer Gesellschaft zur Förderung der angewandten Forschung E.V., 80686 München (DE)
(72) Erfinder: WOLTER, Herbert, 97941 Tauberbischofsheim (DE); SEYFRIED, Mona, 97070 Würzburg (DE); NIQUE, Somchith, 97249 Eisingen (DE)
(74) Vertreter: Strehl Schübel-Hopf & Partner
(86) Internationale Anmeldenummer: PCT/EP2012/060031
(87) Internationale Veröffentlichungsnummer: WO 2012/163911

(56) Entgegenhaltungen:
- WO-A1-03/070198
- US-B2- 7 041 709

## Beschreibung

Die vorliegende Erfindung betrifft Kieselsäure(hetero)polykondensate, umfassend erste, über Kohlenstoff an Silicium gebundene Gruppen, die mindestens eine polymerisierbare C=C-Doppelbindung aufweisen, sowie zweite, ebenfalls über Kohlenstoff an Silicium gebundene Gruppen, die entweder Sulfonat- oder Sulfatgruppen aufweisen, sowie Polymere, die durch die Polymerisation der genannten Doppelbindungen gewonnen werden können.

Polymerisierbare, organische Verbindungen mit Säuregruppen sind für medizinische Produkte zum Erreichen gewünschter Materialeigenschaften wie Benetzung, Ätzwirkung, Komplexierung und damit Haftung auf biologischen Grenzflächen wichtige Bestandteile. Dentaladhäsive basieren auf solchen konventionellen, monomeren Verbindungen, weisen allerdings noch einige erhebliche Defizite auf. Ein wesentliches Problem dabei ist, das die Ätzwirkung im Rahmen der Self-etch-Applikation oftmals nicht ausreicht, um die notwendigen retentiven Strukturen zu realisieren, die für die Haftung und damit einen langlebigen Verbund zwischen Zahngewebe und Restaurationsmaterial erforderlich sind. Somit ist oftmals ein vorheriger separater Ätzschritt mit einem Ätzgel nicht vermeidbar, was wiederum die Fehleranfälligkeit und die Behandlungskosten erhöht. Hinsichtlich der zunehmenden Anforderungen hinsichtlich Biokompatibilität (verwiesen sei auf die Allergiediskussion in Verbindung mit dentalen Monomeren) bieten obige Systeme ebenfalls keine Lösung an. Da die Komponenten des Adhäsivs bei einer Restauration den Zahnwurzeln sowie Blutgefäßen am nächsten kommen, ist es aus toxikologischer Sicht von besonderem Interesse, monomerfreie Systeme bereitzustellen.

In der Patentanmeldung DE 44 16 857 C1 werden Carbonsäure-funktionalisierte (Meth)acrylatalkoxysilane beschrieben. Sie zeichnen sich durch eine Vielfalt von Möglichkeiten aus, die Eigenschaften der daraus resultierenden anorganisch-organischen Verbundpolymere zu variieren bzw. einzustellen. Bedingt durch die enthaltenen Carbonsäuregruppen ergeben sich zusätzliche Reaktionsmöglichkeiten (z. B. Glasionomerreaktionen) sowie eine verbesserte Haftung auf anorganischen Oberflächen. Die Ätzwirkung (s. Self-etch-Applikation) einer Carbonsäuregruppe ist aber bei weitem nicht so groß wie die einer S-OH-Funktion. Entsprechendes gilt für die in EP 1 377 628 B1 beschriebenen phosphonsäurebasierten Systeme. Mit hybridpolymerbasierten Systemen kann im Rahmen der wünschenswerten Self-etch-Applikationen daher bisher kein ausreichend stabiler Verbund zwischen Zahngewebe und Restaurationsmaterial erzielt werden.

Für eine Reihe von Anwendungszwecken wie die Stabilisierung wässriger Silikate oder die Herstellung von elektroviskosen Flüssigkeiten, Emulgatoren, Detergentien oder Schaumbildnern wurden monomere oder kondensierte Silane entwickelt, die Sulfonat- oder Sulfatgruppen enthalten. So beschreibt US 6,777,521 Siliconsulfat-Polymere, die durch die Umsetzung entsprechender Epoxyverbindungen mit Metallsulfat erhältlich sind. Aus US 3,328,449 sind sulfopropylierte organofunktionelle Silane und Siloxane bekannt, die mit Hilfe der Umsetzung von Sultonen erhalten werden können. US 4,777,277 offenbart Organosiloxansulfosuccinate, in denen ein sulfonierter Bernsteinsäureester mit dem Sauerstoffatom der Estergruppe über eine Alkylengruppe an ein Siliciumatom angebunden ist. US 4,503,242 zeigt in Beispiel 1 die Herstellung eines hydrolytisch kondensierbaren Bis-sulfosuccinatamids eines Diaminosilans, erhalten durch die Umsetzung der freien Carbonsäure des entsprechenden Succinatamids mit Natriumsulfit. Ein Silan, das eine Sulfonatgruppe und eine Hydroxygruppe an einem Alkylenoxyalkylenrest des Siliciums trägt, ist in US 5,427,706 offenbart.

Die Verwendung von rein organischen Monomeren, die sowohl eine terminale Sulfonatgruppe als auch eine ungesättigte olefinische Gruppe tragen, für das gleichzeitige Ätzen und Grundieren ("priming") von Zähnen schlägt US 2002/0119426 A1 vor. Auch US 6,759,449 B2 offenbart Dentalkleber-Zusammensetzungen, die sowohl eine organisch polymerisierbare (Meth)Acrylsäure- als auch eine saure Gruppe tragen. Dabei wird zwischen Sulfonatgruppen und Phosphonat- oder anderen sauren Gruppen hinsichtlich der Verwendbarkeit der Verbindungen und ihrer Eigenschaften nicht unterschieden. Gleiches gilt für US 2003/0055124 A1; nur für darin gezeigte (Meth)Acrylamido-phosphonsäuren, nicht aber für ebenfalls aufgezeichnete, entsprechende Sulfonsäuren werden Angaben zur Herstellung gemacht. Eine weitere Anmeldung im Wesentlichen derselben Erfindergruppe, US 2008/0194730, schlägt wiederum die Verwendung von selbstätzenden polymerisierbaren N-substituierten (Meth)Acrylsäureamid-Monomeren für Dentalkomposite vor, die zusätzlich eine saure Einheit tragen, ausgewählt unter Phosphonsäure- und Sulfonsäure-Einheiten. N-Methacryloylaminoalkylsulfonsäuren können nach den Erläuterungen der EP 1 421 927 A1 als selbstätzende Primer für dentale Zwecke eingesetzt werden.

Dentalkleber-Zusammensetzungen aus sauer polymerisierbaren Nanoteilen in einer wässrigen Phase offenbart DE 102 06 451 A1. Die Nanoteilchen bestehen aus Siloxanen, an denen sowohl saure als auch organisch polymerisierbare Gruppen gebunden vorliegen. Die sauren Gruppen können entweder Phosphonat- oder Sulfonatgruppen sein; individuelle spezifische Vorteile für die eine oder die andere Gruppe werden nicht genannt. Das einzige Anwendungsbeispiel nennt einen spezifischen Haftungswert eines Dentalklebstoffs aus einem phosphonsäurehaltigen Material an einer Zahnoberfläche. Ein Verfahren zum Herstellen sulfonatgruppenhaltiger Silane oder Siloxane ist weder allgemein noch hinsichtlich der gezeichneten Verbindungen angegeben.

US 7,041,709 B2 offenbart Kieselsäure(hetero)polykondensate aus Silanen, die sowohl eine (Meth)Acrygruppe als auch eine Sulfonat- oder Sulfatgruppe tragen.
Es besteht ein Bedarf an organisch polymerisierbaren Kieselsäure(hetero)polykondensaten überlegenen Eigenschaften für die Anwendung insbesondere im Dentalbereich. Besonders relevant sind hier eine verbesserte Haftung und/oder eine verbesserte Ätzfunktion und/oder eine Anpassung der optischen Eigenschaften für das kosmetische Erscheinungsbild. Hier Abhilfe zu schaffen, ist die Aufgabe der vorliegenden Erfindung.

Die vorliegende Erfindung ist in den angehängten Patentansprüchen definiert.

In Lösung der Aufgabe werden Kieselsäure(hetero)polykondensate bereitgestellt, die sowohl organisch polymerisierbare Gruppen, insbesondere (Meth)Acrylgruppen, als auch Sulfat- oder Sulfonatgruppen aufweisen. Dabei konnte überraschend festgestellt werden, dass in allen hergestellten Materialien die Sulfonat- bzw. Sulfatgruppe eine wesentlich stärker ätzende Wirkung besitzt als eine Phosphonatgruppe in vergleichbarer Position.

Die erfindungsgemäßen Kieselsäure(hetero)polykondensate umfassen erste, über Kohlenstoff an Silicium gebundene Gruppen, die mindestens eine polymerisierbare C=C-Doppelbindung aufweisen, sowie zweite, ebenfalls über Kohlenstoff an Silicium gebundene Gruppen, die entweder Sulfonat- oder Sulfatgruppen aufweisen. Es handelt sich dabei zumindest formal um Cokondensate aus mindestens einem Silan mit einem über ein Kohlenstoffatom gebundenen Rest, der eine organisch polymerisierbare C=C-Doppelbindung trägt, insbesondere einem (Meth)Acrylrest, und mindestens einem Silan mit einem über ein Kohlenstoffatom gebundenen Rest, der eine Sulfonat- oder Sulfatgruppe, insbesondere der Formel -(O)_{d}-SO₃M mit d = 0 oder 1 und M = Wasserstoff oder ein einwertiges Metallkation oder der entsprechende Anteil eines mehrwertigen Metallkations, trägt. Ausgenommen vom Schutzbereich sind solche Cokondensate, in denen die C=C-Doppelbindungen ausschließlich durch (Meth)Acrylester verwirklicht sind, die in Form einer Methylenacrylestergruppe an die über Kohlenstoff an Silicium gebundenen Gruppen angebunden sind, weil zum ersten die Estergruppe des jeweiligen Methacrylesters nicht hydrolysestabil ist, so dass in unerwünschter Weise freie Alkohol-Moleküle entstehen können, während gleichzeitig die Anzahl von Methacrylsäuregruppen ggf. unkontrolliert zunimmt, und zum zweiten die Doppelbindung aufgrund sterischer Umstände weniger zugänglich ist, denn sie befindet sich nicht im Außenbereich der Silyleinheit, an die sie gebunden ist, so dass sie von ihren Reaktionspartnern weniger gut erreicht werden kann. Außerdem betrifft ein Aspekt der Erfindung einen sehr bequemen Einbau der ggf. vorhandenen (Meth)Acrylgruppen, was dadurch realisiert werden kann, dass diese in Form der freien bzw. einer aktivierten Säure umgesetzt, d.h. an die Kondensate oder deren Silan-Vorläufer angebunden werden. Dies hat zur Folge, dass die (Meth)Acrylgruppe als (Meth)Acrylsäureester, -amid oder-thioester in die Strukturen eingebaut vorliegt.

Der Ausdruck "Cokondensate" soll erfindungsgemäß alle solchen Kondensate umfassen, die bei einer Hydrolyse der Si-O-Si-Bindungen mindestens zwei unterschiedliche Silane liefern würden. Voranstehend wurden diese deshalb "formal" als Cokondensate bezeichnet, weil sie sich auch dadurch herstellen lassen, dass ein einziges Silan hydrolytisch kondensiert wird und nachträglich nur ein Teil der über Kohlenstoff an Siliciumatome gebundenen Gruppen modifiziert wird, wie weiter unten ausführlich dargelegt.

Die erfindungsgemäß einzusetzende organisch polymerisierbare C=C-Doppelbindung ist in einer Reihe von Ausführungsformen eine aktivierte Doppelbindung. Als "aktivierte C=C-Doppelbindungen" sind Gruppen mit solchen Doppelbindungen zu verstehen, in deren Nachbarschaft sich eine elektronenziehende Gruppe befindet, derart, dass ein Angriff durch eine -NHR-Gruppe (ein nucleophiler Angriff) möglich ist. Besonders bevorzugte Beispiele für derartige Reste sind Acrylate und Methacrylate, die entsprechend den vorstehenden Erläuterungen in Form von (Meth)acrylsilylestern, -amiden oder-thioestern vorliegen, d.h. in einer Form, in der die Acrylgruppe mit dem Organosilylrest verestert/amidiert/thioverestert vorliegt.

Statt des Ausdrucks "aktivierte C=C-Doppelbindungen" wird vorliegend an manchen Stellen auch der Ausdruck "aktive C=C-Doppelbindungen" verwendet.

Als Beispiel für organisch polymerisierbare Doppelbindungen, die nicht aktiv bzw. nicht aktiviert sind, seien die Vinyl-, die Allylgruppe sowie in einem Ring befindliche Doppelbindungen wie diejenige der Norbornengruppe genannt. Auch diese Gruppen befinden sich sterisch in einem äußeren Bereich der jeweiligen Silane, so dass sie sich leicht polymerisieren lassen.

Unter dem Ausdruck "organisch polymerisierbar" ist einerseits die Möglichkeit der Polyaddition der Doppelbindungen, andererseits aber auch die Polymerisation durch die Addition von addierbaren Resten wie Thio- oder Aminogruppen zu verstehen. So kann beispielsweise eine Norbornengruppe einer Thiol-En-Addition unterworfen werden.

Das Wort bzw. der Wortbestandteil "(Meth)Acryl..." soll die jeweiligen Methacryl- und Acrylverbindungen gleichermaßen umfassen. Die (Meth)Acrylreste können insbesondere Bestandteil eines (Meth)Acrylsäureesters, -thioesters oder -amids sein. (Meth)Acrylsäureamidreste sind gegenüber den sonstigen (Meth)Acrylresten wegen ihrer besseren Hydrolysebeständigkeit bevorzugt.

Der Ausdruck "Sulf(on)at" umfasst die Sulfonat- und die Sulfatgruppe. Die Ausdrücke "Sulfonatgruppe" und "Sulfatgruppe" umfassen die jeweiligen Säuren und Salze.

Die Polykondensate der Erfindung können in vielen verschiedenen Ausführungsformen vorliegen und nach unterschiedlichen Varianten herstellbar sein. Allen Varianten ist gemeinsam, dass sie in der Regel durch das bekannte Sol-Gel-Verfahren erzeugt werden. Es entstehen dabei Kieselsäurepolykondensate, die häufig auch als ORMOCER®e bezeichnet werden. Die Kondensationsreaktion kann in Gegenwart weiterer Silane der Formel SiR*ₐR**₄₋ₐ erfolgen, die aus dem Stand der Technik in sehr großer Zahl bekannt sind. R* bedeutet darin eine hydrolysierbare Gruppe, die das Einkondensieren des Silans in das Netzwerk ermöglicht, während R** ein beliebiger, nicht kondensierbarer Rest ist. R* kann hier OH oder eine C₁-C₁₀-Alkoxygruppe, stärker bevorzugt eine C₁-C₄-Alkoxygruppe und ganz besonders bevorzugt Methoxy oder Ethoxy sein. R* kann je nach Bedarf aber auch ein Halogenid wie Cl, Wasserstoff, Acyloxy mit vorzugsweise 2 bis 5 Kohlenstoffatomen, Alkylcarbonyl mit vorzugsweise 2 bis 6 Kohlenstoffatomen oder Alkoxycarbonyl mit vorzugsweise 2 bis 6 Kohlenstoffatomen sein. In manchen Fällen kann R* auch NR² mit R² gleich Wasserstoff, Alkyl mit vorzugsweise 1-4 Kohlenstoffatomen oder Aryl mit vorzugsweise 6-12 Kohlenstoffatomen sein.

Die Silane beider Varianten werden dabei in geeigneter Weise so ausgewählt, dass sich mit ihnen der gewünschte Kondensationsgrad erzielen lässt. So werden bis zu drei Reste, gegebenenfalls aber nur zwei Reste der Silylgruppe ausgewählt unter Hydroxygruppen oder - bevorzugt - hydrolysierbaren Gruppen. Solche Gruppen werden allgemein als Netzwerkbildner bezeichnet. Bevorzugt handelt es sich bei diesen um Alkoxy-, Aryloxy- oder Aralkoxygruppen, insbesondere C₁-C₁₀-Alkoxygruppen, stärker bevorzugt C₁-C₄-Alkoxygruppen und ganz besonders bevorzugt Methoxy oder Ethoxy. Je nach Bedarf können in speziellen Fällen als hydrolytisch kondensierbare Gruppen aber statt dessen bzw. zum Teil, jeweils unabhängig voneinander, Halogenide wie Cl, Wasserstoff, Acyloxy mit vorzugsweise 2 bis 5 Kohlenstoffatomen, Alkylcarbonyl mit vorzugsweise 2 bis 6 Kohlenstoffatomen oder Alkoxycarbonyl mit vorzugsweise 2 bis 6 Kohlenstoffatomen sein ausgewählt werden, sofern diese nicht mit den Reaktionen interferieren, die zur Herstellung der erfindungsgemäßen Kondensate benötigt werden. In Einzelfällen können stattdessen Gruppen der Bedeutung NR² mit R² gleich Wasserstoff, Alkyl mit vorzugsweise 1-4 Kohlenstoffatomen oder Aryl mit vorzugsweise 6-12 Kohlenstoffatomen eingesetzt werden.

Darüber hinaus können die Silane auch Gruppen enthalten, die als Netzwerkwandler bezeichnet werden. Bei diesen handelt es sich um Gruppen, die selbst keinen Einfluss auf die Bildung des Kondensats haben, dessen Eigenschaften aber modifizieren können. Hierbei handelt es sich vorzugsweise um substituierte oder unsubstituierte, geradkettige, verzweigte oder mindestens einen Cyclus aufweisende Alkyl-, Aryl-, Arylalkyl-, Alkylaryl- oder Alkylarylalkylgruppen; in Einzelfällen können jedoch auch entsprechende Alkenyl-, Arylalkenyl- oder Alkenylarylgruppen vorhanden sein. Bevorzugt sind Alkyl-, Aryl- oder Aralkylgruppen, insbesondere C₁-C₁₀-Alkylgruppen, stärker bevorzugt C₁-C₄-Alkylgruppen und ganz besonders bevorzugt Methyl oder Ethyl.

In Ausnahmefällen kann ein einziges Silan zwei über Kohlenstoff an das Silicium gebundene Gruppen aufweisen, die entweder beide mindestens eine polymerisierbare C=C-Doppelbindung aufweisen oder beide eine Sulfonat- oder Sulfatgruppe aufweisen.

Bei Vorhandensein von drei hydrolysierbaren Gruppen / Hydroxygruppen entsteht ein dreidimensionales Netzwerk, während Silane mit zwei Hydroxygruppen / hydrolysierbaren Gruppen Ketten und/oder Ringe ausbilden. Da die meisten der für die Erfindung einsetzbaren Silane nur eine über Kohlenstoff an Silicium gebundene Gruppe aufweisen, die entweder eine polymerisierbare C=C-Doppelbindung oder eine Sulfonat- oder Sulfatgruppe trägt, weisen sie im Falle des Vorhandenseins von nur zwei hydrolysierbaren Gruppen / OH-Gruppen in der Regel eine der oben genannten Gruppen auf, die als Netzwerkwandler bezeichnet werden.

Es kann wünschenswert sein, zusätzliche Metallverbindungen für die Einkondensierung in das anorganische Netzwerkt vorzusehen. Hierfür kommen insbesondere hydrolytisch kondensierbare Verbindungen von Metallen der III. und der IV. Hauptgruppe sowie der III. bis VI. Nebengruppe in Betracht, z.B. des Bors, des Aluminiums, des Titans des Germaniums, des Zirkoniums oder des Zinns. Diese Metallverbindungen sind in großer Zahl bekannt. In diesen Fällen entsteht ein Kieselsäure(hetero)polykondensat, bei dem die genannten Metallatome in das Si-O-Si-Netzwerk eingebunden sind. Die zusätzlichen Metallverbindungen sind häufig Alkoxyverbindungen; In spezifischen Ausführungsformen der Erfindung können die anderen Metallverbindungen jedoch ebenfalls ihrerseits reaktive Gruppen aufweisen. Besonders interessant für die vorliegende Erfindung sind dabei Komplexe, die ihrerseits (Meth)Acrylgruppen tragen, da diese bei einer nachfolgenden organischen Polymerisation in das organische Netzwerk mit eingebunden werden können.

Die Herstellung der Polykondensate lässt sich prinzipiell in zwei Gruppen einteilen: Gemäß Variante (A) werden unterschiedliche Silane bereitgestellt oder erzeugt, von denen mindestens eines eine Sulfonat- oder eine Sulfatgruppe und mindestens ein zweites eine organisch polymerisierbare Gruppe trägt, die mindestens eine C=C-Doppelbindung aufweist. Gemäß Variante (B) wird dagegen zuerst ein Polykondensat aus einem oder mehreren Silanen erzeugt, das noch nicht alle erfindungsnotwendigen Gruppen trägt, und sodann wird auf der Stufe des Polykondensats eine Modifikation durchgeführt, die das erfindungsgemäße Polykondensat ergibt.

Die Variante (A) lässt sich mit Hilfe einer Vielzahl von teils käuflichen, teils durch den Fachmann leicht herstellbaren Ausgangs-Silanen durchführen. Beispielsweise kann ein Methacrylsilylester unaufwändig durch Umsetzung eines entsprechenden Silylalkohols mit Methacrylsäurechlorid oder eines entsprechenden Silylepoxids mit Methacrylsäure erhalten werden. Weitere Ausgangs-Silane sind beispielsweise in DE 40 11 044 C1 und DE 44 16 857 C1 beschrieben. Von Vorteil ist es, dass auch solche Silane eingesetzt werden können, die mehr als eine C=C-doppelbindungshaltige Gruppe und/oder mehr als eine Sulf(on)atgruppe aufweisen, die sich vorzugsweise an demselben über ein Kohlenstoff an das Silicium gebundenen Rest befinden. Ein Beispiel hierfür ist die nachstehende (Reaktion 7):

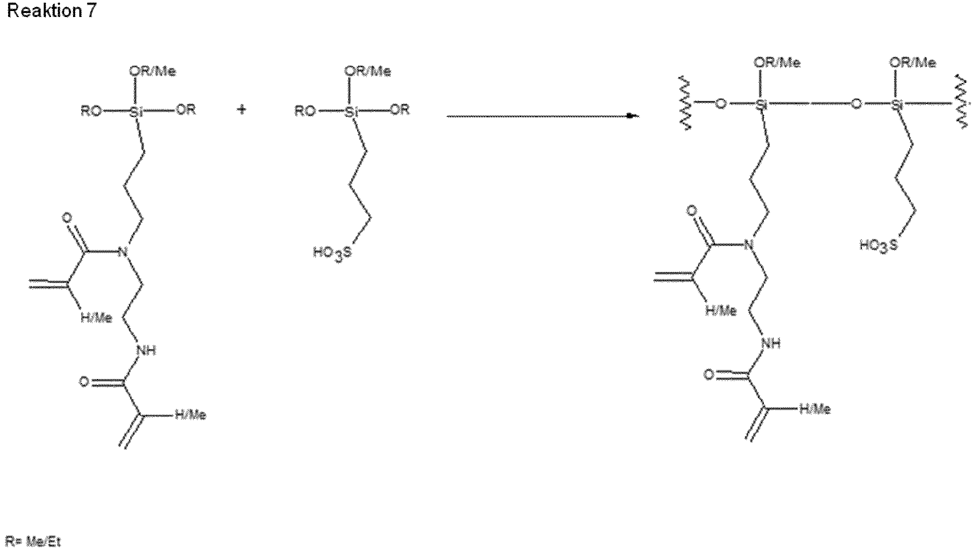

Der hier verwendete N-methacryl-modifizierte Methacrylsilylamid-Rest lässt sich dadurch herstellen, dass ein Silan mit einer über ein Kohlenstoffatom an das Si-Atom gebundenen Kohlenwasserstoffgruppe bereitgestellt wird, die eine primäre und eine sekundäre Aminogruppe trägt und mit aktivierter Methacrylsäure (z.B. Methacrylchlorid oder-anhydrid) umgesetzt wird. Varianten lassen sich herstellen, indem anstelle des primären Amins eine Hydroxygruppe oder eine Thiolgruppe vorhanden ist und/oder dass anstelle des sekundären Amins eine Seitengruppe mit einem primären Amin, einer Hydroxygruppe oder einer Thiolgruppe vorhanden ist. Beispiele für geeignete Aminosilane sind die Verbindungen (Aminoethyl-aminomethyl)phenylethyltrimethoxysilan, N-(2-Aminoethyl)-3-aminopropylmethyldimethoxysilan, N-(2-Aminoethyl-3-aminopropyl)trimethoxysilan, N-2-Aminoethyl-3-aminopropyltris(2-ethylhexoxy)silan, 6-(Aminohexylaminopropyl)-trimethoxysilan, N-(N'-(2 Aminoethyl)aminoethyl)-3-aminopropyl-trimethoxysilan, N-(N'-(2 Aminoethyl)aminoethyl)-3-aminopropyl-methyldimethoxysilan, N-(N'-(2 Aminoethyl)aminoethyl)-3-aminopropyl-triethoxysilan, N-(N'-(2 Aminoethyl)aminoethyl)-3-aminopropyl-methyldiethoxysilan, N-(N'-(2 Aminoethyl)aminoethyl)-3-aminopropyl-trimethylsilan, N-(N'-(2 Aminoethyl)aminoethyl)-3-aminopropyl-tris (methoxyethoxyethoxy)silan. Analoge Verbindungen mit entsprechenden Hydroxy- oder Thiolgruppen sind beispielsweise in EP 0 779 890 A1 offenbart. Auch die Kohlenwasserstoffgruppe kann einen anderen Aufbau haben als in den oben gezeigten Beispielen.

Es ist offensichtlich, dass statt einer Silylgruppe mit zwei Funktionen, die sich mit (Meth)Acrylsäure umsetzen lassen, auch eine solche mit drei oder noch mehr solchen Funktionen eingesetzt werden kann. Die jeweils erhältlichen (Meth)Acryl-Silane ermöglichen aufgrund der jeweils vorhandenen Anzahl an (Meth)Acrylgruppen eine Einstellbarkeit der Dichte des bei der späteren Polymerisation erzielbaren organischen Netzwerks. Selbstverständlich können die Anzahl und die chemische Struktur der Reste RO in Reaktion 7 wie voranstehend definiert ausgewählt werden; die Angaben "R= Me/Et", in denen Me Methyl und Et Ethylbedeutet, sind rein beispielhaft.

Das Silylsulfonat lässt sich beispielsweise durch Umsetzung von Allylsulfonat mit einem Hydridosilan oder durch Umsetzung von Natriumsulfit mit einem Epoxysilan erzeugen. Auch das Sulf(on)at-Silan kann beliebig modifiziert werden. Beispielsweise kann die Sulfonsäuregruppe des entsprechenden Silans über eine durch Sauerstoffatome und/oder Aminogruppen unterbrochene Kohlenstoffkette mit dem Siliciumatom verbunden sein. Solche Silane lassen sich durch Umsetzung eines epoxidhaltigen Silans mit Natriumsulfit, Aminoethansulfonat, Methylaminoethansulfonat oder dgl. erhalten, wie beispielsweise aus der weiter unten diskutierten Reaktion 6 ersichtlich, in der diese Reaktionen allerdings erst nach der hydrolytischen Kondensation der jeweiligen Silane durchgeführt wird. Wird anstelle von Natriumsulfit Natriumsulfat eingesetzt wie beispielsweise in US 6,777,521 beschrieben, erhält man ein entsprechendes Sulfat.

Gemäß Variante (B) wird ein Kondensat entweder aus nur einem Silan erzeugt, das jedoch nicht alle erfindungswesentlichen Gruppen trägt, wobei nachträglich nur ein Teil der über Kohlenstoff am Silicium gebundenen Gruppen mit einem geeigneten Reaktionspartner modifiziert wird (Variante B1), meist durch Einführung einer Sulfonsäure- oder Sulfonatgruppe, oder es werden zur Herstellung des Kondensats zwei oder sogar mehr Silane eingesetzt, von denen mindestens eines nachträglich modifiziert wird (Variante B2), ebenfalls meist durch Einführung einer Sulfonsäure- oder Sulfonatgruppe.

Die Variante (B1) hat den Vorteil, dass es möglich ist, durch Variation der Menge an zugegebener Sulfonsäureverbindung das Verhältnis der Gruppen mit polymerisierbarer Doppelbindung zu den Gruppen mit Sulf(on)at beliebig einstellen zu können. Ein Beispiel für diese Variante zeigt die nachstehende Reaktion 5:

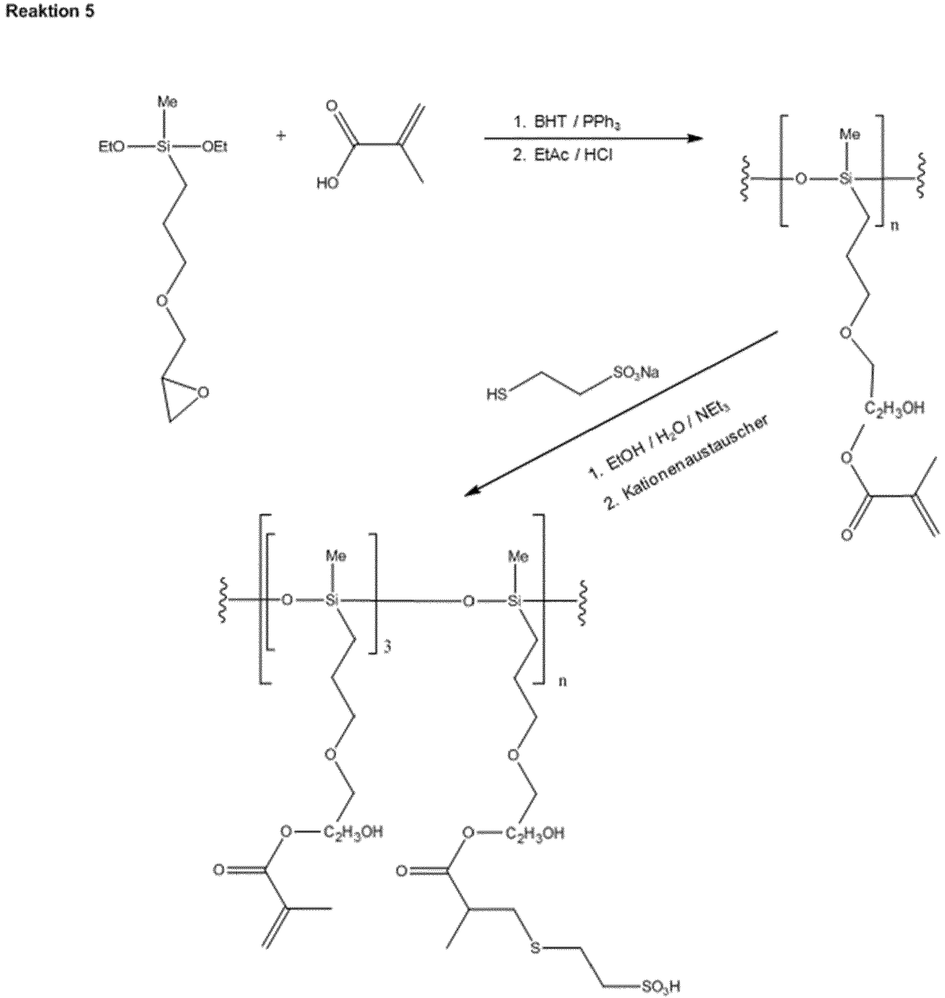

Auch bei der Herstellung von Kondensaten gemäß Variante (B) können natürlich bereits bekannte Verbindungen eingesetzt oder bekannte Reaktionsabfolgen genutzt werden, da sich sowohl bekannte (Meth)Acrylsilane als auch bekannte, Sulfonatgruppen enthaltende Silane verwenden lassen. Die Herstellung von Kondensaten wie in Stufe 1 der Reaktion 5 gezeigt ist bereits aus DE 44 16 857 bekannt. Ein Teil der Methacrylatreste wird anschließend zur Anbindung einer Sulfonatgruppe genutzt, wofür im Beispiel eine Thioalkansulfonsäure eingesetzt wird. In diesem konkreten Beispiel ist es natürlich wichtig, dass die Thioalkansulfonsäure im Unterschuss eingesetzt wird, um einen Anteil an (Meth)Acrylatgruppen zu erhalten. Der Vorteil einer Reaktionsweise wie in diesem Beispiel liegt darin, dass das Verhältnis von (Meth)Acrylgruppen zu Sulfonsäure- bzw. Sulfatgruppen im Kondensat beliebig ausgewählt werden kann. Ein weiterer Vorteil liegt darin, dass die bei der Ringöffnung des Epoxids entstandene Hydroxygruppe erhalten bleiben kann, da sie nicht für die Anbindung des (Meth)Acrylats genutzt werden muss. Sie kann daher für andere Zwecke genutzt werden, z.B. zur Erhöhung der Matrixhydrophilie der Kieselsäurepolykondensate oder zur Anbindung weiterer reaktiver Gruppen, beispielsweise einer weiteren (Meth)Acrylatgruppe.

Selbstverständlich lässt sich diese Reaktion in beliebiger Weise durch einfachen Austausch von Substituenten durch andere Substituenten, wie aus dem Stand der Technik bekannt, modifizieren. So kann anstelle eines Epoxy-Silans z.B. ein Aminosilan eingesetzt werden, so dass die Methacrylgruppe in Form des Methacrylamids vorliegt. Wie oben zu Variante (A) beschrieben, kann das als Ausgangsmaterial verwendete Silan natürlich auch mehrere reaktive Gruppen enthalten, die sich mit aktivierter (Meth)Acrylsäure umsetzen lassen, oder es wird von vorneherein ein Silan eingesetzt, das eine (nicht aktivierte) Doppelbindung trägt, beispielsweise ein Vinyl- oder Allylsilan. Eine andere Variation ist der Austausch der Methacrylsäure durch ein Brücken-Ringsystem wie eine Norbornengruppe, die durch Umsetzung mit Cyclopentadien erhalten werden kann, siehe die nachfolgende Umsetzung:

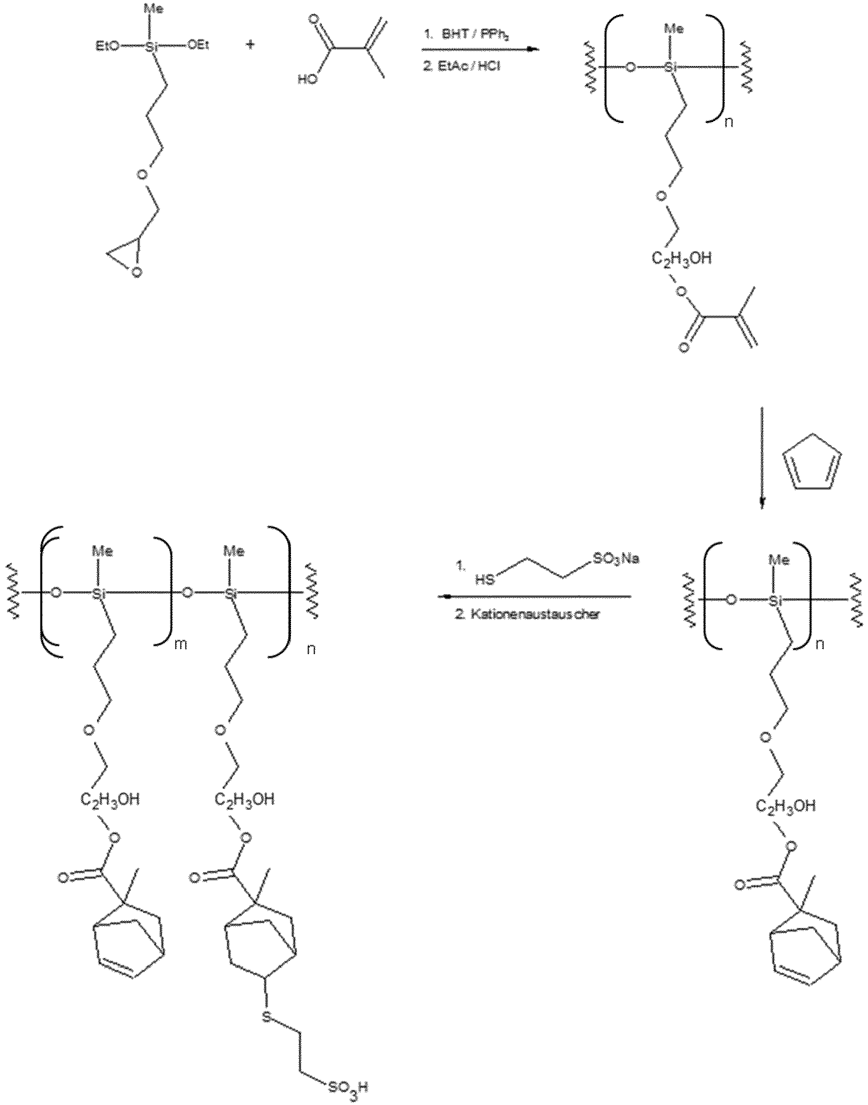

Bezüglich einsetzbarer Norbornensilane und verwandter Verbindungen kann auch auf DE 196 27 198 A1 verwiesen werden. So kann u.a. der oben im Reaktionsschema gezeigte Norbornenring selbstverständlich ggf. substituiert sein; auch kann anstelle des Norbornen-Restes (d.h. des Bicyclo[2.2.1]heptenrestes) ein Bicyclo[2.2.2]octen-Rest vorhanden sein. Weiterhin kann der doppelbindungshaltige Fünfring des kondensierten Systems ein Sauerstoffatom enthalten, wenn die (Meth)Acrylgruppe mit Furan anstelle von Cyclopentadien umgesetzt wird.

Die Variante (B2) lässt sich anhand des nachfolgenden Schemas erläutern:

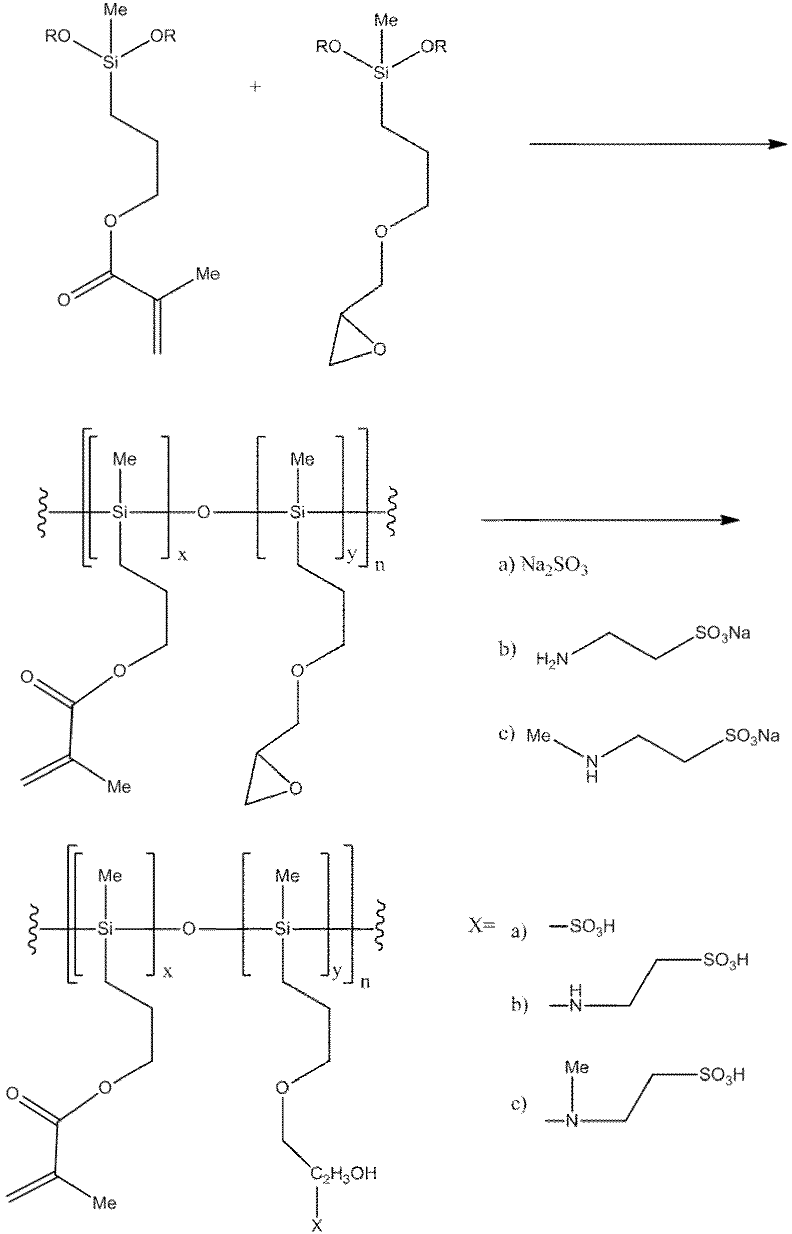

Dieses Reaktionsschema zeigt die Cokondensation zweier Silane, von denen eines eine (Meth)Acrylgruppe, das andere einen gespannten Heteroring trägt. Die hydrolytische Kondensation kann in bekannter Weise so geführt werden, dass der Heteroring geschlossen bleibt. An den Heteroring wird nach der Kondensationsreaktion eine Sulfonatgruppe angebunden. Geschieht dies gemäß b) mit Hilfe der Anbindung über eine Aminogruppe, lässt sich die Reaktion leicht - und ebenfalls in bekannter Weise - so führen, dass die Aminogruppe nicht oder kaum an der Doppelbindung der Methacrylgruppe angreift.

Gemäß dem Reaktionsschema entsteht ein erfindungsgemäßes Kondensat wie voranstehend beschrieben. Bei einer solchen Reaktionsführung kann das Verhältnis von (Meth)Acrylgruppen zu Sulf(on)atgruppen durch das Verhältnis der eingesetzten Silane zueinander bestimmt werden. Im Übrigen können auch dieselben bzw. vergleichbare Reaktionstypen wie weiter oben gezeigt verwendet werden.

Anstelle einer Methacrylgruppe wie hier dargestellt können selbstverständlich andere C=C-doppelbindungshaltige Gruppen eingesetzt werden, die in beliebiger Weise an das jeweilige Siliciumatom angebunden sind. Eine kleine Auswahl zeigt das nachstehende Schema:

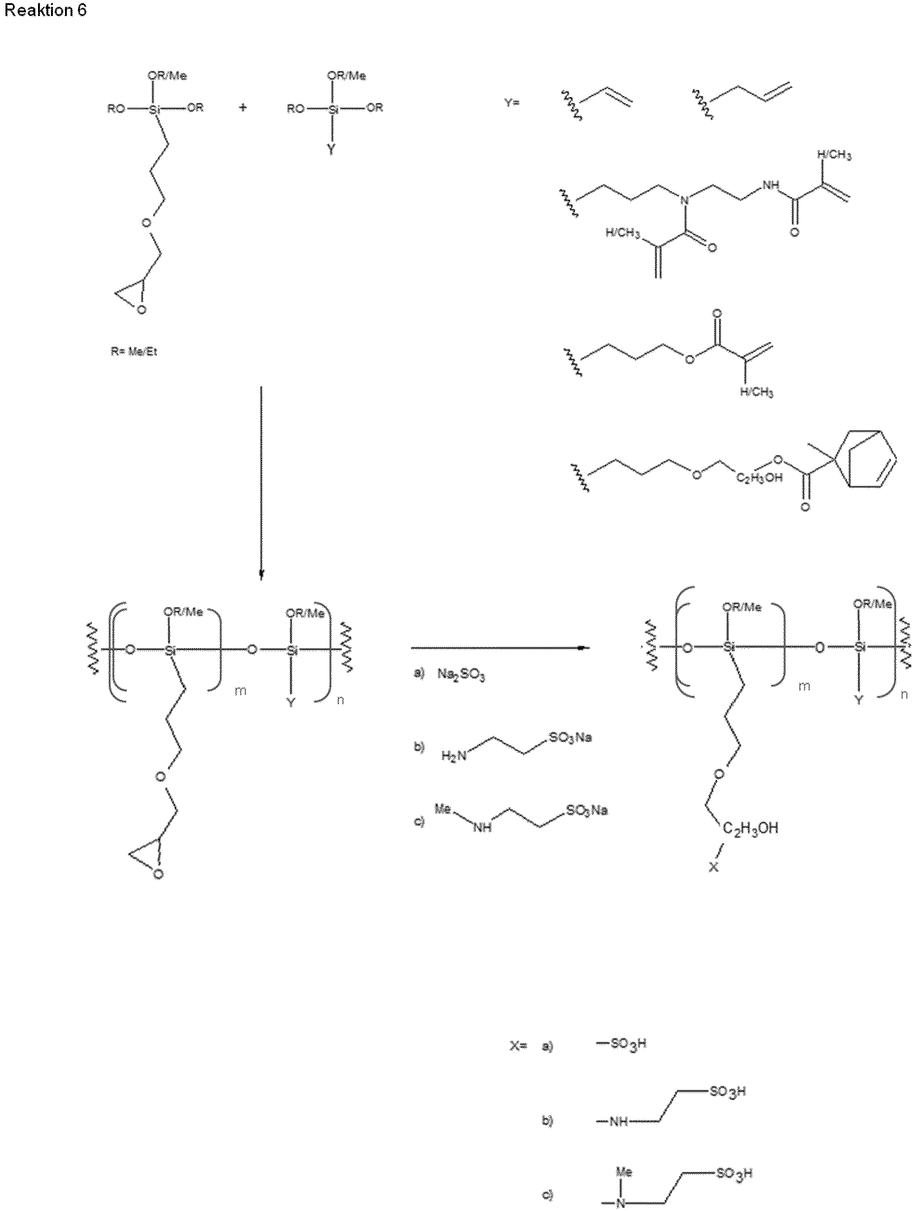

Eine weitere Alternative bietet der Einsatz eines Silans, das wie in Reaktion 5, Schritt 1 gezeigt durch Umsetzung eines Epolysilans mit Methacrylsäure gewonnen wurde. Bei dieser Alternative folgt Schritt 2, die hydrolytische Kondensation, nicht unmittelbar auf die Herstellung des Silans, wie in Reaktion 5 gezeigt, sondern das methacrylierte Silan wird mit weiterem Epoxysilan vermischt, worauf die Mischung von Epoxysilan mit Methacrylsilan der hydrolytischen Kondensation unterworfen wird, wie in Reaktion 6 dargestellt.

Die erfindungsgemäßen Kieselsäure(hetero)polykondensate können weitere funktionellen Gruppen tragen, die ihnen für spezielle Anwendungen vorteilhafte Eigenschaften verleihen können. An vorderster Stelle sind hierbei Carbonsäuregruppe und Hydroxygruppen zu nennen. Beispiele für das Vorhandensein von Hydroxygruppen finden sich in den voranstehenden Reaktionen 5, 6 und im Schema zur Variante B2. Diese lassen sich beispielsweise dadurch erhalten, dass ein zusätzliches Silan, das eine solche Gruppe trägt, mit den übrigen Ausgangssilanen cokondensiert wird. Statt dessen kann ein Silan verwendet werden, das zusätzlich zu dieser Gruppe eine polymerisierbare C=C-Doppelbindung und/oder eine Sulf(on)atgruppe aufweist, und zwar ggf. an derselben, über Kohlenstoff an das Silicium gebundenen Gruppe. Entsprechende Silane werden weiter unten beschrieben.

Alle erfindungsgemäßen Kieselsäure(hetero)polykondensate, unabhängig davon, ob gemäß Variante (A) oder einer der Varianten (B) hergestellt, können zusätzlich unter Verwendung mindestens eines Silans hergestellt sein, das einen über Kohlenstoff an das Silicium gebundenen Rest besitzt, der sowohl eine organisch polymerisierbare C=C-Doppelbindung als auch eine Sulf(on)atgruppe aufweist. Dieses Silan lässt sich beispielsweise mit der Formel (I)

R¹ₐR²_{b}SiZ_{4-a-b} (I)

darstellen, worin R¹ ein hydrolytisch kondensierbarer Rest ist, R² substituiertes oder unsubstituiertes, geradkettiges, verzweigtes oder mindestens einen Cyclus aufweisendes Alkyl, Aryl, Arylalkyl, Alkylaryl oder Alkylarylalkyl ist, ausnahmsweise aber stattdessen ein entsprechendes Alkenyl sein bzw. ein solches umfassen kann, dessen Kohlenstoffkette in allen Fällen gegebenenfalls durch -O-, -S-, -NH-, -S(O)-, -C(O)NH-, -NHC(O)-, -C(O)O- -C(O)S, - NHC(O)NH- oder C(O)NHC(O)-Gruppen unterbrochen sein kann, die ggf. in beide möglichen Richtungen weisen können, Z ein Rest ist, in welchem mindestens eine (Meth)Acrylgruppe und mindestens entweder eine Sulfonat- oder eine Sulfatgruppe unmittelbar oder mittelbar über eine unsubstituierte oder substituierte Kohlenwasserstoffgruppe am Siliciumatom gebunden vorliegen, a 1, 2 oder 3 ist, b 0, 1 oder 2 ist und a+b zusammen 2 oder 3 sind. Insbesondere ist es bevorzugt, wenn die Reste Z weiterhin jeweils mindestens eine Hydroxy- oder eine Carbonsäuregruppe oder einen davon abgeleiteten Ester oder ein solches Salz aufweisen.

In einer Reihe von bevorzugten Ausführungsformen lassen sich die Silane der Formel (I) mit der nachstehenden Formel (la) darstellen: 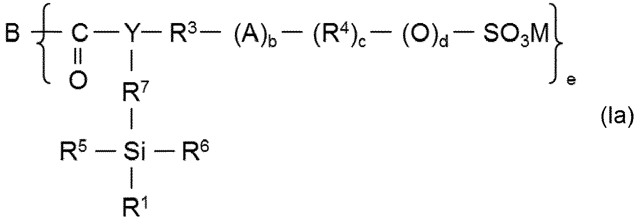 worin
R¹ ein hydrolytisch kondensierbarer Rest ist,
R³ ein, unsubstituiertes oder mit einer funktionellen Gruppe substituiertes, geradkettiges, verzweigtes oder mindestens einen Cyclus aufweisendes Alkylen ist,
A eine Verknüpfungsgruppe darstellt,
R⁴ ein gegebenenfalls durch O, S, NH oder NR⁸ unterbrochenes und/oder gegebenenfalls funktionell substituiertes Alkylen bedeutet,
M Wasserstoff oder ein einwertiges Metallkation oder der entsprechende Anteil eines mehrwertigen Metallkations, vorzugsweise ausgewählt unter Alkali- und Erdalkalikationen, insbesondere unter Na, K, ½Ca, 1/2Mg, oder Ammonium ist,
R⁵ und R⁶ unabhängig voneinander entweder die Bedeutung wie R¹ haben oder substituiertes oder unsubstituiertes, geradkettiges, verzweigtes oder mindestens einen Cyclus aufweisendes Alkyl, Aryl, Arylalkyl, Alkylaryl oder Alkylarylalkyl sind, ausnahmsweise stattdessen aber auch ein entsprechendes Alkenyl, Arylalkenyl oder Alkenylaryl sein können,
R⁷ eine über ein Kohlenstoffatom an das Siliciumatom gebundene Kohlenwasserstoffgruppe ist, wie sie weiter oben definiert wurde ,
R⁸C₁-C₆-Alkyl oder (Meth)Acryl ist,
B Vinyl, 2-Allyl oder, im Falle von e > 1, ein organischer Rest mit e Vinylgruppen ist, die jeweils an eine in der geschweiften Klammer befindliche Gruppe gebunden vorliegen
Y ein Stickstoffatom, -O-CH=, -S-CH= oder -NH-CH= ist, wobei jeweils das Sauerstoffatom, das Schwefelatom bzw. die NH-Gruppe eine Bindung zur benachbarten C(O)-Gruppe aufweist,
b = 0 oder 1 ist
c = 0 oder 1 ist
mit der Maßgabe, dass für die Kombination von Y gleich einem Stickstoffatom, b = 0, c = 0 und d = 0 der Rest R³ Ethylen ist sowie der Maßgabe, dass für die Kombination von Y gleich einem Stickstoffatom, b = 0, c = 1 und d = 0 der Rest R⁴ ein durch O, S, NH oder NR⁸ unterbrochenes und gegebenenfalls funktionell substituiertes Alkylen bedeutet,
d = 0 oder 1 ist, und
e = 1, 2 oder 3 ist.

Die Verknüpfungsgruppe A in der Formel (la) wird vorzugsweise ausgewählt unter (gelesen von links nach rechts in der Formel Ia) C(O)NH, NHC(O), NR⁸C(O), C(O)O und OC(O), wobei R⁸ wie oben definiert ist. In Ausnahmefällen kann die Verknüpfungsgruppe A diese Gruppen jedoch statt dessen in zur Leserichtung entgegengesetzter Richtung aufweisen und zusätzlich unter NHC(O)O, NR⁸C(O)O, NHC(O)NH, C(O)NHC(O) und C(O)S ausgewählt sein. Der Rest R⁴ ist in spezifischen Ausführungsformen substituiert mit mindestens einer Hydroxygruppe und/oder mit einem Rest R⁹COOM, worin R⁹ eine chemische Bindung oder ein C₁-C₆-Alkylenrest ist und M Wasserstoff oder ein einwertiges Metallkation oder der entsprechende Anteil eines mehrwertigen Metallkations, vorzugsweise ausgewählt unter Alkali- und Erdalkalikationen, insbesondere unter Na, K, ½Ca, 1/2Mg, oder Ammonium ist.

Mit wenigen Ausnahmen werden die Synthesen zur Herstellung der Silane der Formel (I) bzw. (la) so geführt, dass für die Addition der Sulfonsäure- bzw. Sulfatgruppe an das bereits eine (Meth)Acrylgruppe enthaltende Molekül eine C=C-Doppelbindung zur Verfügung steht. Nach Wahl kann an diese entweder Natriumsulfit oder eine Sulfonsäure mit einem bequem addierbaren Rest wie eine Hydroxy-, Thio- oder Aminoalkansulfonsäure addiert werden. Alternativ kann die Anbindung der Sulfonsäuregruppe auch nach dem umgekehrten Prinzip erfolgen, indem ein Hydroxy-, Thio- oder Aminoalkylsilan mit einer Alkylensulfonsäure zur Reaktion gebracht wird. Bei diesem Verfahren ist natürlich eine Kettenverlängerung durch die Kohlentoffatome der Alkylengruppe unvermeidlich, weshalb die erstere Variante gegenüber der zweiten bevorzugt ist. Schließlich gibt es auch noch die Möglichkeit, die Ringöffnung eines gespannten Heterorings, insbesondere eines Dreirings, mit Sulfit oder einer Hydroxy-, Thio- oder Aminoalkansulfonsäure zu bewirken. Diese Variante hat den Vorteil, dass bei der Ringöffnung eine weitere reaktive Gruppe entsteht, die für die folgende Anbindung der aktivierten (Meth)Acrylsäure genutzt werden kann. Der Dreiring kann alternativ auch mit Hilfe eines Sulfats geöffnet werden; in diesen Fällen erhält man ein sulfatgruppenhaltiges Produkt.

Insgesamt stehen damit erfindungsgemäß drei grundlegende Varianten für die Herstellung der Silane mit der Formel (I) zur Verfügung wie folgt:
Variante (i):
   a. ein Silan mit einer über ein Kohlenstoffatom an das Si-Atom gebundenen Kohlenwasserstoffgruppe wird bereitgestellt, die mindestens zwei funktionelle Gruppen trägt, ausgewählt unter primären Aminen, sekundären Aminen, Hydroxygruppen und Thiolgruppen,
   b. eine erste der beiden funktionellen Gruppen wird mit gegebenenfalls aktivierter (Meth)Acrylsäure und die zweite der beiden funktionellen Gruppen wird mit einer gegebenenfalls aktivierten, zweiten, eine C=C-Doppelbindung aufweisenden und gegebenenfalls mindestens eine weitere Funktionalität aufweisenden Carbonsäure umgesetzt, und
   c. im Anschluss an die genannte Reaktion wird eine sulfonat- oder sulfatgruppenhaltige Verbindung oder ein Sulfit an die C=C-Doppelbindung des mit der zweiten funktionellen Gruppe umgesetzten Carbonsäurerestes addiert, derart, dass an dem mit der ersten der beiden funktionellen Gruppen umgesetzten (Meth)Acrylrest eine solche Addition nicht stattfindet, was sich auf verschiedenen Wegen, z.B. über das molare Verhältnis der miteinander umgesetzten Gruppen, sicherstellen lässt,
      wobei es sich bei der zweiten eine C=C-Doppelbindung aufweisenden Carbonsäure um (Meth)Acrylsäure oder um eine andere doppelbindungshaltige Carbonsäure handeln kann.
Variante (ii):
   a. ein Silan mit einer über ein Kohlenstoffatom an das Si-Atom gebundenen Kohlenwasserstoffgruppe wird bereitgestellt, die mindestens einen reaktionsfähigen Heteroring trägt, ausgewählt unter den Dreiringen Oxacycyclopropyl- (=Epoxy-), Azacyclopropyl und Thiocyclopropyl und cyclischen Carbonaten (letztere können durch Umsetzung eines Epoxyrings mit CO₂, aber auch auf anderen Wegen erhalten werden, wie in DE 44 23811 ausführlich dargestellt),
   b. der Heteroring wird mit einem Sulfit oder einem Sulfat oder einer sulfonat- oder sulfatgruppenhaltigen Verbindung umgesetzt und
   c. zumindest die dabei freiwerdende OH-, SH- bzw. NH₂-Gruppe wird mit gegebenenfalls aktivierter (Meth)Acrylsäure umgesetzt.
Variante (iii)
   a. ein Silan mit einer über ein Kohlenstoffatom an das Si-Atom gebundenen Kohlenwasserstoffgruppe wird bereitgestellt, die eine Aminogruppe oder eine Mercaptogruppe aufweist,
   b. ein Alkenylsulfonat oder ein Sulfon wird mit der Aminogruppe bzw. der Mercaptogruppe zur Reaktion gebracht, und
   c. die in b. entstandene sekundäre Aminogruppe bzw. Thiogruppe wird mit gegebenenfalls aktivierter (Meth)acrylsäure umgesetzt.

Ein Beispiel für die Herstellung eines solchen Silans gemäß Variante (i) sei nachstehend beispielhaft anhand der Reaktion 1 gezeigt:

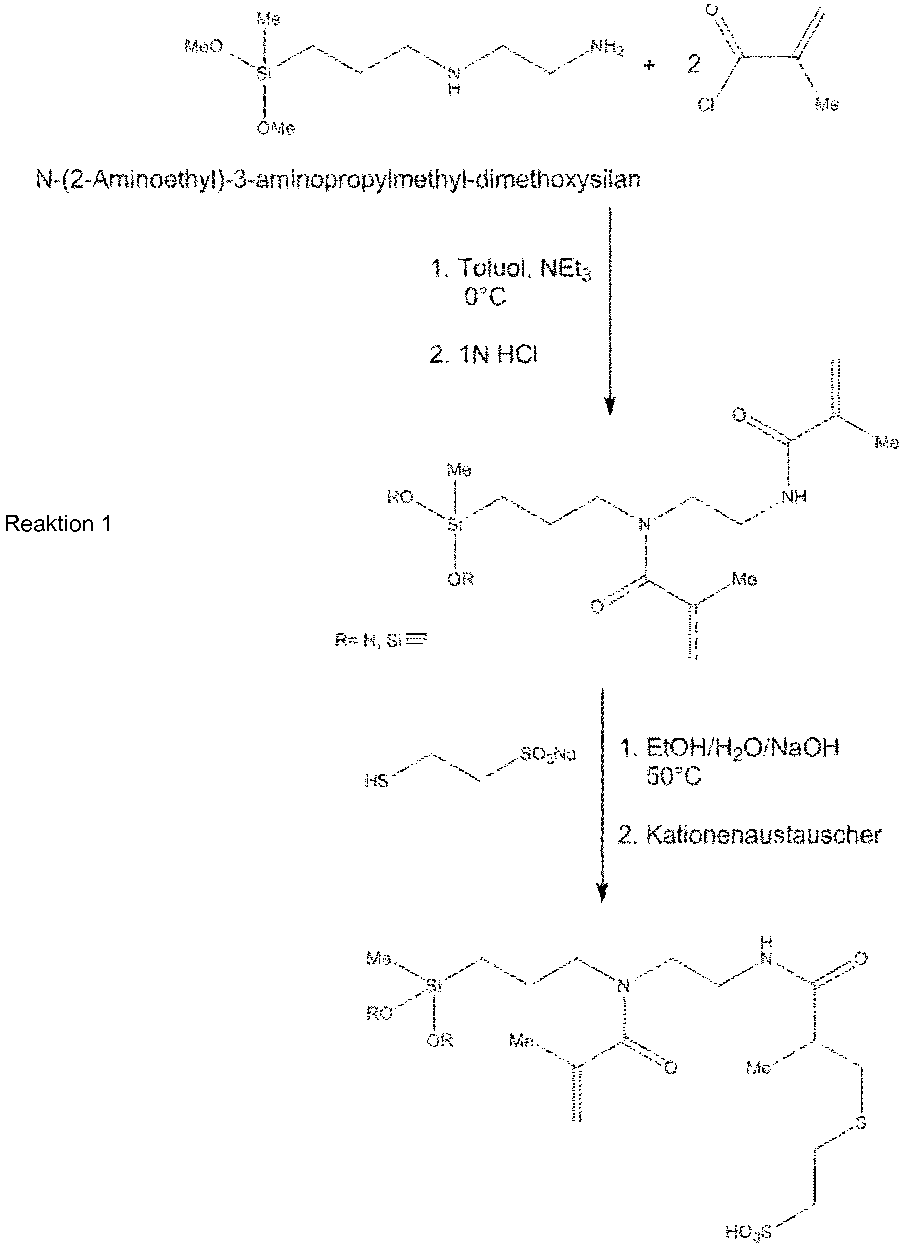

Eine Umsetzung gemäß Variante (ii) sei nachstehend beispielhaft anhand der Reaktion 3 gezeigt:

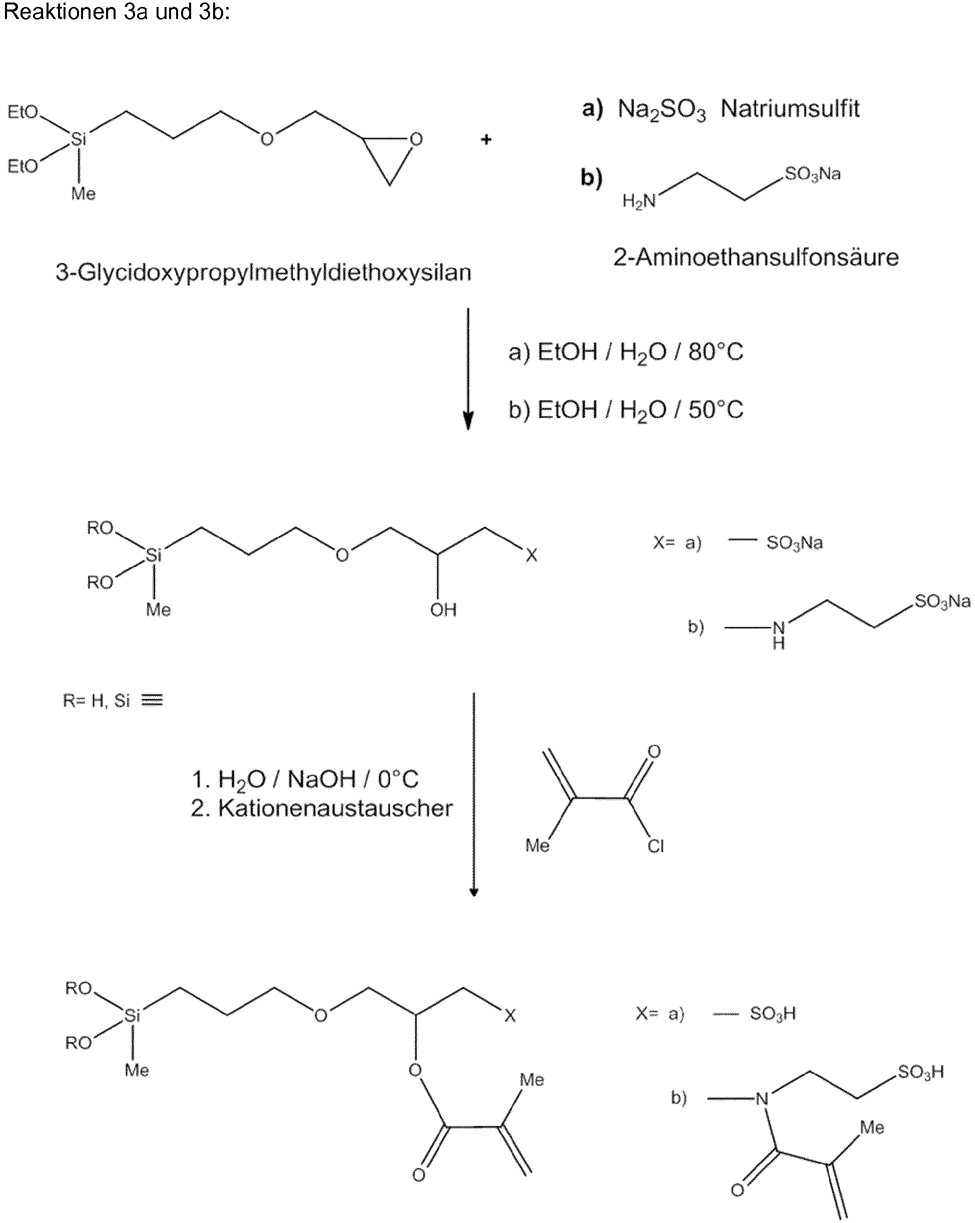

Eine Umsetzung gemäß Variante (iii) sei nachstehend beispielhaft anhand der Reaktion 4 gezeigt:

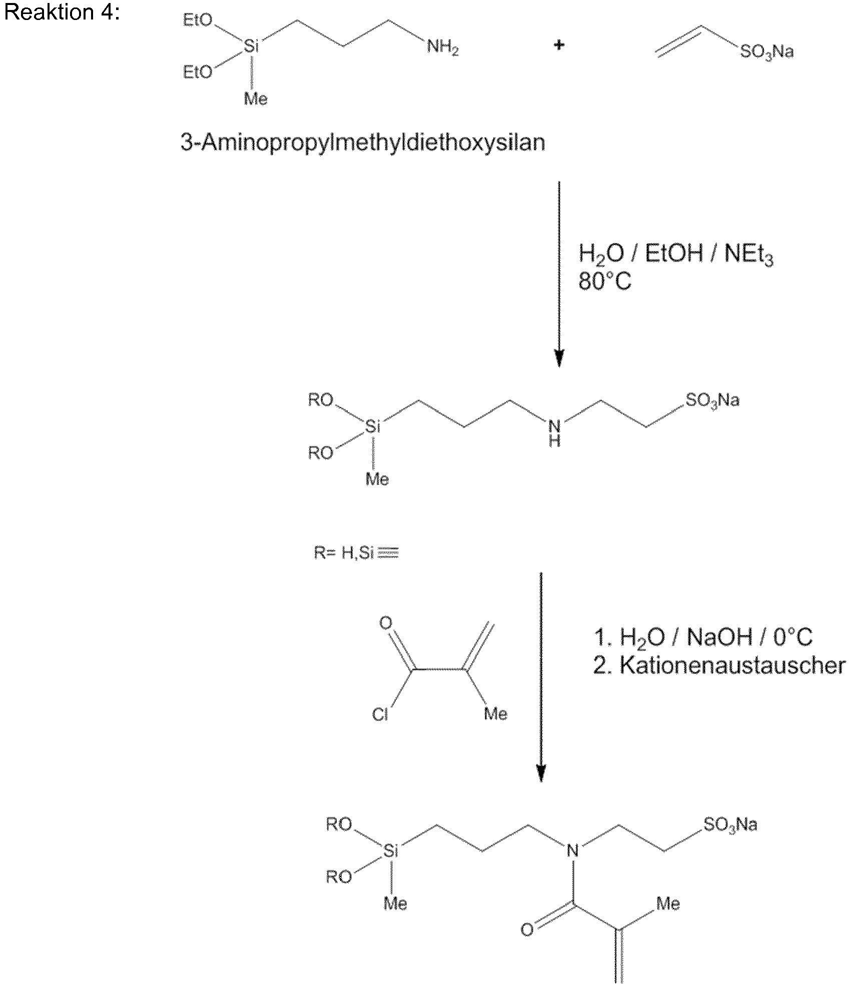

Die voranstehenden Reaktionsbeispiele zeigen Umsetzungen zu Sulfonaten. Durch die Verwendung von Sulfaten wie in US 6,777,521 beschrieben anstelle von Sulfiten in den Umsetzungen gemäß Variante (ii) lassen sich entsprechende Sulfatverbindungen erhalten.

Die Synthesen zur Herstellung der erfindungsgemäßen Kieselsäure(hetero)polykondensate zeichnen sich in allen Varianten durch Einfachheit der Reaktionsführung, eine geringe Anzahl von Arbeitsschritten und gute Ausbeuten aus.

Wie teilweise bereits oben erwähnt, kann in spezifischen Ausführungsformen der Erfindung ein an einem Siliciumatom gebundener Kohlenwasserstoffrest mit mehr als einer Sulfonsäure- oder Schwefelsäuregruppe und/oder mit mehr als einer (Meth)Acrylgruppe substituiert sein. Durch das Vorhandensein von mehr als einer (Meth)Acrylgruppe kann das Netzwerk, das sich beim Polymerisieren der Kondensate bildet, noch engmaschiger werden. In diesem Zusammenhang ist zu erwähnen, dass über den Gehalt an polymerisierbaren Doppelbindungen der E-Modul des späteren organisch polymerisierten Polymer eingestellt werden kann, derart, dass das Polymer mehr oder weniger flexibel und dabei weniger hart oder härter ausfällt. Durch das Vorhandensein von mehr als einer Sulfonsäure- oder Schwefelsäuregruppe wird die Ätzwirkung des Kondensats weiter erhöht.

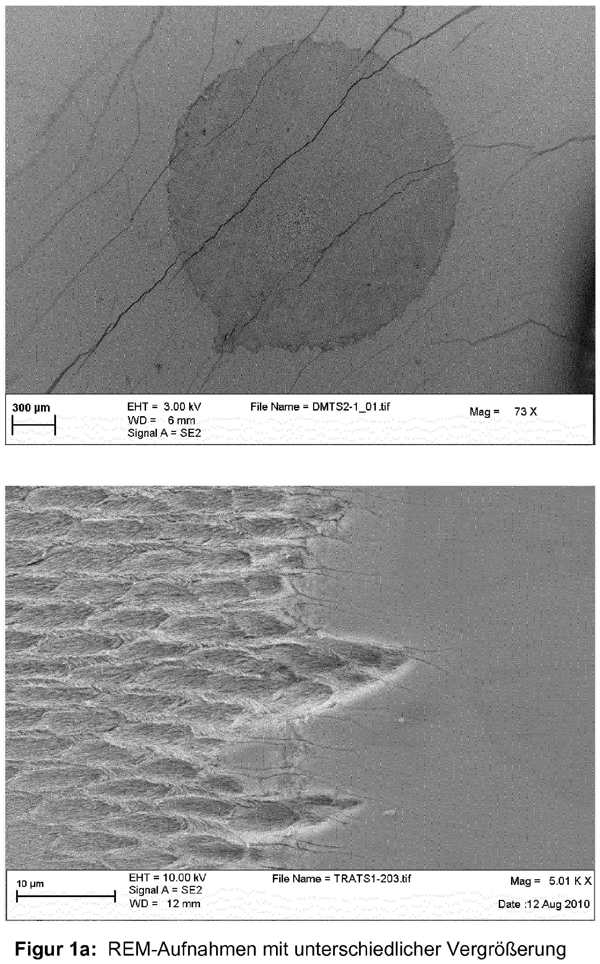

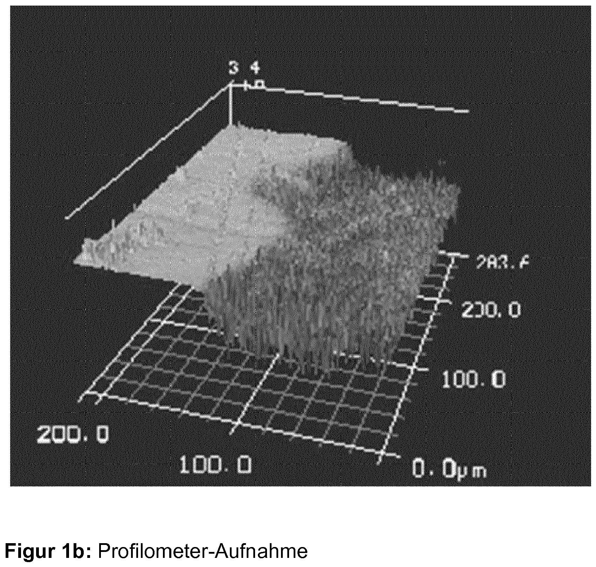

Die Erfinder konnten überraschend feststellen, dass bereits mit geringen Sulfonsäuregehalten eine enorme Ätzwirkung auf dem Zahngewebe beobachtet werden kann Dies lässt sich anhand eines Vergleichs der mittleren Rauheit der Schmelzoberfläche aufzeigen: Polierter Schmelz besitzt eine mittlere Rauheit von etwa 0,21 µm, gemessen mit einem optischen Profilometer der Firma UBM. Mit einem phosphonsäurefunktionalisierten Kieselsäurepolykondensat aus Glycerin-1-methacryloyl-2-(siloxypropyl)carboxymethylphosphonsäure kann man Rauheiten im Bereich von 0,33 erzielen. Mit Kondensaten der erfindungsgemäßen Verbindungen liegt die Rauheit im Bereich von über 0,45µm. Zahnschmelzaufnahmen sind in den **Figuren 1a** **und** **1b** gezeigt.

Die Vielseitigkeit und die gezielte Anpassungsmöglichkeit der erfindungsgemäßen Kieselsäure(hetero)polykondensate und der daraus gebildeten oder geformten Polymere an den jeweiligen Zweck beruht ganz wesentlich auf den aufgezeigten Herstellungsmöglichkeiten. Auf die Verwendung zusätzlicher Monomere, z.B. die Verwendung von Reaktivverdünnern mit dem Ziel einer ausreichenden organischen Vernetzung der Polymere, kann daher häufig verzichtet werden. Dies ist insbesondere dann von Vorteil, wenn die erfindungsgemäßen Kondensate und die daraus erhältlichen Polymere im medizinischen, z.B. dentalen Bereich eingesetzt werden sollen. Denn aus der zunehmenden öffentlichen Diskussion zum Thema ist bekannt, dass (Meth)Acrylat-basierte Monomere im Verdacht stehen, Allergien auslösen zu können.

Überrascht wurden die Erfinder im Übrigen von der Tasache, dass die erfindungsgemäßen Kieselsäurepolykondensate in der Regel wasserlöslich sind, obwohl sie ja eine Vielzahl von (Meth)Acrylatgruppen tragen und eine anorganisch kondensierte Struktur aufweisen. Dies hat für viele Anwendungen enorme Vorteile, wobei an vorderster Stelle medizinische Applikationen zu nennen sind. Denn die Kondensate können in wässrigem Medium angewendet, d.h. in beliebiger Form appliziert werden, ohne dass der Einsatz eines nichtwässrigen Lösungsmittels erforderlich wäre. Aber auch für industrielle Anwendungen sind wasserbasierte Reaktionen immer vorteilhaft, und zwar schon aus Gründen der Arbeitssicherheit und der Umweltverträglichkeit.

Die Möglichkeit, neben der Sulfonsäurefunktion weitere reaktive Gruppen in den Silanen auszubilden, erschließt zusätzliche Möglichkeiten. So haben Sulfonsäure- eine stärkere Ätzwirkung als Carboxylgruppen, während diese komplexbildende Eigenschaften haben. Sofern zusätzliche Hydroxygruppen vorhanden sind, können diese entweder zur Verbesserung der Benetzung am Untergrund oder für weitere Umsetzungen genutzt werden, die die erfindungsgemäßen Kieselsäure(hetero)polysiloxane weiter modifizieren können. Ein Beispiel ist eine Komplexierung oder eine Umsetzung mit einer Dicarbonsäure (die z.B. mit Hilfe entsprechend aktivierter Säuremoleküle bewirkt werden kann).

Neben Polymerisationsgrad und Ätzwirkung besitzen die erfindungsgemäß an den Polykondensaten der Erfindung befindlichen Gruppen und Reste weitere Eigenschaften, die günstig für eine Reihe von Verwendungszwecken sind: Die Sulfonat- bzw. Sulfatgruppe ist ein Ladungsträger, weshalb Verwendungen als Elektrophoresegele, als Materialien für die Elektrotauchlackierung oder als die Leitfähigkeit oder die Antistatik modifizierende Materialien möglich sind. Des Weiteren kann die Gruppe als saurer Katalysator dienen, und zwar zum einen für den Sol-Gel Prozeß (ein späterer Abtrennungsschritt zur Katalysator-Abtrennung kann dann entfallen) und zum anderen im Hinblick auf den späteren Einsatz (eine Beispiel sind mesoporöse Membranen mit Sulfonsäuregruppen, die als Katalysator für chemische Prozesse dienen können). Die Gruppe sorgt ferner für eine gute Löslichkeit in polaren Medien. Insbesondere, aber nicht nur für dentale Zwecke dient sie als haftvermittelnde Gruppe für anorganische, organische sowie hybride Oberflächen. Genauso wie Carbonsäuregruppen kann sie aber auch ionische Bindungen ausbilden, wodurch sich z.B. Alkali-, Erdalkali-, Ammonium-, Ti-, Zr-, Sn-, Ca- und andere geeignete Kationen in Form ihrer Salze in das Polykondensat-Netzwerk einbinden lassen. Hierdurch lassen sich eine Reihe von Modifikation bzw. materialspezifischen Einstellungen erreichen, z.B. hinsichtlich der Röntgenopazität, der Brechzahl oder der Kontakttoxizität. Durch die Sulfonat- bzw. Sulfatgruppen im Material erhält dieses im Übrigen eine antimikrobiellen Wirkung. Aber auch auf ganz anderen Gebieten lässt sich die Erfindung anwenden, weil man mit sulfonat- bzw. sulfatgruppenhaltigen Materialien z.B. protonenleitende Membranen, z.B. für fuel cells, bilden kann. Weiter kommen die Materialien in Betracht z.B. als Ionenaustauscher, als pseudostatische Phase in der elektrokinetischen Chromatographie oder als grenzflächenaktiver Stoff (Tensid).

Insbesondere durch die Kombination der Sulfonat- bzw. Sulfatgruppen und ggf. zusätzlich der -CO₂H-Gruppen mit polymerisierbaren / polyaddierbaren Doppelbindungen in einem Molekül bietet sich der Einsatz im Medizinsektor (spez. Dentalbereich) z. B. als Haftvermittler (monomerfreies Bonding) und als Matrixbestandteil für Zemente an.

Das Mengenverhältnis von C=C-doppelbindungshaltigem Silan zu sulf(on)athaltigem Silan im erfindungsgemäßen Kondensat ist im Grunde nicht kritisch. So kann die Zahl der über Kohlenstoff an Silicium gebundenen C=C-doppelbindungshaltigen Reste zur Zahl der über Kohlenstoff an Silicium gebundenen sulf(on)athaltigen Reste beispielsweise im Bereich von 10:1 bis 1:10 liegen. Besonders bevorzugt ist ein Verhältnis von 3:1 bis 1:1, und häufig wird man ein Verhältnis von etwa 1:1 wählen. Verhältnisse, in denen die Zahl der C=C-doppelbindungshaltigen Reste relativ zur Zahl der sulf(on)athaltigen Gruppen überwiegt, sind in einer Reihe von Fällen bevorzugt, weil dann ein besonders dichtes organisches Netz erzeugt werden kann. Das gleiche Ziel kann man natürlich auch dadurch erreichen, dass über Kohlenstoffatome an das Silicium gebundene Gruppen eingesetzt werden, die mehr als eine C=C-Doppelbindung tragen.

Durch Einsatz von beliebigen Füllstoffen (Partikel, Fasern) wie z. B. den in DE 196 43 781, DE 198 32 965, DE 100 18 405, DE 104 10 38, DE10 2005 018 351, DE 10 2005 061 965 sowie in DE 10 2005 053 721 beschriebenen Partikeln oder von SiO₂-Partikeln in Kombination mit den erfindungsgemäßen Kieselsäure(hetero)polykondensaten werden entsprechende Komposite erhalten. SiO₂-Partikel kann man beispielsweise nach bekannten Sol-Gel-Verfahren erhalten; sie können eine sehr enge Durchmesserverteilung aufweisen. Diese oder auch anders zusammengesetzte Nanopartikel können auf ihrer Oberfläche modifiziert, z.B. silanisiert sein, um ihre Oberflächeneigenschaften an diejenigen der Matrix anzupassen.

Die Komposite sind plastisch verarbeitbar und zeichnen sich durch mögliche sehr hohe Füllstoffgehalte (s. Nanohybridkomposite) in Kombination mit einer ausgezeichneten Verarbeitbarkeit aus. Somit können unterschiedliche Eigenschaften in weiten Bereichen sowohl bei den resultierenden Silanen, Harzsystemen, Matrixsystemen sowie den gefüllten Systemen (Kompositen) eingestellt und den Erfordernissen angepasst werden.

Besonders bevorzugt ist der Einsatz der Erfindung im Dentalbereich, d. h. z. B. als Adhäsiv. Hier kann insbesondere für die Herstellung von Dentalkompositen das durch hydrolytische Kondensation aus den erfindungsgemäßen Silanen erhaltene Kieselsäure(hetero)polykondensat vor der organischen Aushärtung mit einem oder mehreren Zusatz- und/oder Füllstoffen gemischt werden. Wesentlicher Bestandteil in dieser Hinsicht sind nanopartikuläre Füllstoffe oder eine Kombination solcher Füllstoffe verschiedener Größe oder verschiedener Zusammensetzung wie oben erwähnt, ggf. in Kombination mit weiteren, bekannten Füllstoffen wie partikulären Dentalgläsern, z.B. Ba-Sr-Aluminiumborosilikaten.

Der Füllstoff kann je nach gewünschtem Anwendungsbereich in sehr unterschiedlichen Gesamtmengen zugesetzt werden. So benötigen Adhäsive nur relativ kleine Füllstoffmengen. Auch Fissurenversiegler, Zahnhalsbeschichter und dergleichen enthalten in der Regel einen Anteil von weniger als 50 Gew.%, z.B. 1-50 Gew.-% und bevorzugt von ca. 1 bis 20 Gew.-% an Füllstoff. In anderen Fällen kann der Füllstoff gegebenenfalls z.B. in einem Anteil von 50 Gew.-% des Komposits oder sogar deutlich darüber und insbesondere in einem Anteil von 70 bis 90 Gew.-% des Komposits vorliegen, wenn höher- oder hochgefüllte Komposite benötigt werden, z.B. für Füllungen oder dergleichen,

Je nach dem vorgesehenen speziellen Verwendungszweck können dem Kieselsäure(hetero)-polykondensat bzw. dem Komposit der Erfindung geeignete Additive wie Initiatoren, Färbemittel (Farbstoffe oder Pigmente), Oxidationsinhibitoren, Polymerisationsinhibitoren (für die Vermeidung einer vorzeitigen Polymerisation), Verlaufsmittel, UV-Absorber, Stabilisatoren, mikrobiozide Wirkstoffe oder dergleichen zugesetzt werden, wie es dem Fachmann bekannt ist. Beispiele für Polymerisationsinitiatoren sind Initiatoren für die radikalische Polymerisation, und zwar für die thermische Härtung wie Peroxide (z.B. Dibenzoylperoxid) oder Photoinitiatoren wie Benzophenon, Campherchinon oder Kombinationen von α-Diketonen mit Aminen als Reduktionsmittel, wie beispielsweise aus der DE 199 03 177 C2 bekannt. Für die duale Aushärtung von radikalisch und kationisch polymerisierbaren Systemen können insbesondere Diaryliodonium- oder Triarylsulfoniumsalze zugesetzt werden, für die die vorgenannte Druckschrift ebenfalls Beispiele angibt.

Das gefüllte Dentalkomposit (d.h. das organisch noch nicht vernetzte, gefüllte Harz) kann, nachdem es für den vorgesehenen Zweck angewendet wurde, in geeigneter Weise organisch vernetzt und damit ausgehärtet werden. Dem dient vor allem eine organische Polymerisation der (Meth-)Acrylatgruppen. Diese ist eine radikalische Polymerisation, die üblicherweise unter Zusatz von Radikalstartern wie den oben erwähnten und ggf. bekannten Aktivatoren unter Belichtung mit z. B. sichtbarem Licht (Blaulicht; Dentalstrahler) d. h. photochemisch, thermisch oder redoxinduziert, aber auch im Rahmen von 2-Komponenten-Reaktionen oder anaerob erfolgt. Die Kombination von Selbsthärtung mit z. B. photoinduzierter bzw. thermischer Härtung ist ebenfalls möglich.

Der Einsatz der erfindungsgemäßen Kieselsäure(hetero)polykondensate erstreckt sich jedoch nicht nur auf für dentale Zwecke angepasste Komposite, sondern u. a. auch auf die Verwendung in Form von Bulkmaterialien, (Zahn-)Zementen, Klebstoffen, Vergussmassen, Beschichtungsmaterialien, Haftvermittlern, Bindemitteln für keramische Partikel (keramische Formgebungsverfahren), zur Herstellung bzw. Primung von Füllstoffen und Fasern, Einsatz im Reaktionsextruder und dergleichen für die verschiedensten Zwecke (insbesondere für medizinische-, aber auch für (mikro)optische- und (mikro)elektronische Anwendungen). Die Kondensate lassen sich mit Hilfe einer Vielzahl von Verfahren in strukturierte Flächen oder Körper überführen, beispielsweise durch Siebdruck, Tintenstrahldruck, Laserdirektschreiben, Photolithographie oder Zwei- oder Mehrphotonenpolymerisation.

Nachstehend soll die Erfindung anhand von Beispielen näher erläutert werden.

### Beispiel 1: Herstellung eines Kieselsäurepolycokondensats gemäß Variante A

Zu einer Lösung aus 3,4 g (0,01mol) N,N' Dimethacryloyl-3-(2-aminoethylamino)-propylmethyldimethoxysilan in Aceton/Wasser (Verhältnis 1:1) wurden 2,5 g (0,01 mol) 3-Trimethoxysilylpropylsulfonsäure gegeben und bei 40 °C gerührt, bis die Kondensation abgeschlossen war. Anschließend wurden die flüchtigen Bestandteile unter Vakuum entfernt.

### Beispiel 2: Herstellung eines Kieselsäurepolycokondensats gemäß Variante B1 (Reaktion 5)

Stufe 1: In 377.8 g (1.521 mol) Diethoxy(3-glycidyloxypropyl)methylsilan wurden 3.93 g (0.015 mol) Triphenylphosphin und 0.316 g (0.06 Gew.%) Butylhydroxytoluol gelöst. Anschließend wurde die Lösung auf 85°C erhitzt und 142.05 g (1.65 mol) Methacrylsäure über 1.5 h hinzu getropft. Nach 24 h unter Rückfluss wurde das Produkt in Ethylacetat gelöst und mit 1N Salzsäure auf pH 1-2 eingestellt. Nach 3 d wurde die Lösung mit 1 N Natriumhydroxid Lösung bis zu einem pH - Wert von 12 gewaschen und anschließend die flüchtigen Bestanteile unter Vakuum entfernt.
Stufe 2: Zu einer Lösung aus 7.78 g (0.008 mol) Produkt der Stufe 1, 0.68 g NaOH in 40 mL Ethanol wurde bei 50°C 1.33 g (0.008 mol) Natrium 2-Mercaptoethansulfonat in 40 mL H₂O hinzu getropft. Nach 19 h wurde Ethanol bei 40°C unter Vakuum entfernt und die wässrige Lösung zweimal mit Ethylacetat gereinigt. Die wässrige Phase wurde mit einem Kationenaustauscher behandelt und die flüchtigen Bestandteile anschließend unter Vakuum entfernt. Das Produkt ist in Wasser wieder löslich.
   mittlere Rauheit Rₐ= 0.45 µm (auf Schmelz)

### Beispiel 3: Herstellung eines Kieselsäurepolycokondensats gemäß Variante B2:

Stufe 1: Zu einer Lösung aus 2,2 g (0,01 mol) Methacryloxypropyltrimethoxysilan in 50 mL Ethylacetat wurden 2,2 g (0,01mol) 3-Glycidoxypropylmethyldimethoxysilan gegeben. Nach Zugabe eines gängigen Katalysators, wie etwa Ammoniumfluorid wurde das Reaktionsgemisch bei 40 °C gerührt, bis die Kondensation abgeschlossen war. Nach üblicher Aufarbeitung, z.B. Ausschütteln mit Wasser, wurden die flüchtigen Bestandteile unter Vakuum entfernt.
Stufe 2: Das Cokondensat aus Stufe 1 wurde in 20 mL Ethanol gelöst und auf 80°C erhitzt. Eine Lösung aus (0,01 mol) Natriumbisulfit in 20 mL Wasser wurde hinzugetropft und das Reaktionsgemisch unter Rückfluss für 4 h gerührt. Nach Abdampfen von Ethanol wurde die wässrige Phase mit Ethylacetat gereinigt, mit einem Kationenaustauscher behandelt und die flüchtigen Bestandteile anschließend unter Vakuum entfernt.

### Beispiel 4: Herstellung eines Silans mit einer über Kohlenstoff am Silicium gebundenen Gruppe, die eine C=C-Doppelbindung und eine Sulfonatgruppe trägt

Stufe 1: 5.11 g (0.024 mol) N-(2-Aminoethyl)-3-aminopropylmethyldimethoxysilan wurde in 5.21 g Triethylamin und 30 mL Toluol gelöst und auf 0°C temperiert. Anschließend wurden 5.0 mL (0.051 mol) Methacrylsäurechlorid in 30 ml Toluol hinzu getropft. Das Reaktionsgemisch wurde 3 h bei Raumtemperatur gerührt. Das Gemisch wurde zentrifugiert und die erhaltene Lösung mit 1N Salzsäure auf pH 1-2 eingestellt. Nach 24 h wurden die flüchtigen Bestandteile unter Vakuum entfernt
Stufe 2: 3.92 g (0.013 mol) des Produkts aus Stufe 1 wurden in 30 mL Ethanol gelöst, die Lösung mit Natriumhydroxid auf pH 10 eingestellt und auf 60°C erwärmt. Anschließend wurden 1.93 g (0.015 mol) Natrium 2-Mercaptoethansulfonat gelöst in 40 mL H₂O zugetropft und für 4 h gerührt. Ethanol wurde unter Vakuum entfernt und die wässrige Lösung mit einem Kationenaustauscher behandelt. Die flüchtigen Bestandteile wurden unter Vakuum entfernt. Das Produkt ist ein wasserlöslicher Feststoff.
   mittlere Rauheit Rₐ= 0.58 µm (auf Schmelz)

### Beispiel 5: Herstellung eines Silans mit einer über Kohlenstoff am Silicium gebundenen Gruppe, die eine C=C-Doppelbindung und eine Sulfonatgruppe trägt

Stufe 1: 8.69 g (0.042 mol) N-(2-Aminoethyl)-3-aminopropylmethyldimethoxysilan
   wurden in 50 mL Ethylacetat gelöst und auf 50°C erwärmt. Eine Lösung aus 4.23 g (0.043 mol) Maleinsäureanhydrid in 30 mL Ethylacetat wurde zugetropft und 19 h gerührt. Das Gemisch wurde zentrifugiert und der Rückstand zweimal mit Ethylacetat gereinigt und unter Vakuum getrocknet.
Stufe 2: 6.06 g (0.021 mol) des Produkts der Stufe 1 wurden in 5 mL Wasser und 1.72 g NaOH gelöst und auf 0°C temperiert. 2.1 mL (0.021 mol) Methacrylsäure-chlorid wurden unter starkem Rühren langsam hinzu getropft und für 5 h bei 50°C gerührt. Die flüchtigen Bestandteile wurden anschließend unter Vakuum entfernt.
Stufe 3: 9.82 g (0.021 mol) des Produkts der Stufe 2 wurden in 20 mL Wasser gelöst und auf 60° erwärmt. Anschließend wurden 2.61 g (0.021 mol) Natriumsulfit unter Rühren zugetropft und für 24 h gerührt. Die wässrige Lösung wurde mit einem Kationenaustauscher behandelt und die flüchtigen Bestandteile unter Vakuum entfernt. Das Produkt lässt sich in Wasser wieder lösen. mittlere Rauheit Rₐ= 0.48 µm (auf Schmelz)

### Beispiel 6: Herstellung eines Silans mit einer über Kohlenstoff am Silicium gebundenen Gruppe, die eine C=C-Doppelbindung und eine Sulfonatgruppe trägt

Stufe 1: In 30 mL H₂O wurden 5.04 g (0.040 mol) Natriumsulfit gelöst und auf 80°C erhitzt. Eine Lösung aus 9.96 g (0.040 mol) 3-Glycidoxypropylmethyldiethoxysilan in 10 mL Ethanol wurde hinzu getropft und für 3 h unter Rückfluss gerührt.
   Nach Abdampfen von Ethanol wurde die wässrige Phase mit Ethylacetat gereinigt und die flüchtigen Bestandteile unter Vakuum entfernt.
Stufe 2: 5.04 g (0.016 mol) des Produkts der Stufe 1 wurde in 10 mL Wasser und 2.79 g (0.070 mol) NaOH gelöst und auf 0°C temperiert. 4.0 mL (0.016 mol) Methacrylsäurechlorid wurden anschließend zugetropft und das Reaktionsgemisch für 4 h bei 30°C gerührt. Die Lösung wurde mit Ethylacetat gereinigt, die wässrige Phase mit einem Kationenaustauscher behandelt und die flüchtigen Bestandteile anschließend unter Vakuum entfernt. Das Produkt ist wasserlöslich.

## Patentansprüche

1. Kieselsäure(hetero)polykondensat aus mindestens einem ersten Silan mit einem über ein Kohlenstoffatom an Silicium gebundenen Rest (1), der eine organisch polymerisierbare C=C-Doppelbindung trägt, und mindestens einem zweiten Silan mit einem über ein Kohlenstoffatom an Silicium gebundenen Rest (2), der eine Sulfonat- oder Sulfatgruppe der Formel -(O)_{d}-SO₃M mit d = 0 oder 1 und M = Wasserstoff oder ein einwertiges Metallkation oder der entsprechende Anteil eines mehrwertigen Metallkations trägt, wobei das erste Silan einen Rest (2) nicht trägt und das zweite Silan einen Rest (1) nicht trägt, wobei in dem Fall, dass die organisch polymerisierbare C=C-Doppelbindung in einer (Meth)Acrylgruppe enthalten ist, diese Gruppe als (Meth)Acrylsäureester, -amid oder -thioester vorliegt, mit Ausnahme von Polykondensaten, in denen die C=C- Doppelbindungen ausschließlich durch Methacrylester gebildet werden, die in Form einer Methylenacrylestergruppe an die über Kohlenstoff an Silicium gebundenen Gruppen angebunden sind.

2. Kieselsäure(hetero)polykondensat nach Anspruch 1, worin die organisch polymerisierbare C=C-Doppelbindung eine Vinylgruppe oder Teil einer Allyl-, Acryl-, Methacryl-, ggf. substituierten Bicyclo[2.2.1]heptengruppe, ggf. substituierten Bicyclo[2.2.2]octengruppe oder einer ggf. substituierten Oxabicyclo[2.2.1]heptengruppe ist.

3. Kieselsäure(hetero)polykondensat nach Anspruch 1 oder 2, umfassend weiterhin mindestens ein Silan mit einem über ein Kohlenstoffatom an Silicium gebundenen Rest, der sowohl eine organisch polymerisierbare C=C-Doppelbindung als auch eine Sulfonat- oder Sulfatgruppe trägt.

4. Kieselsäure(hetero)polykondensat nach einem der Ansprüche 1 bis 3, umfassend über ein Kohlenstoffatom an Silicium gebundene Reste, die jeweils mindestens eine Carbonsäuregruppe oder einen davon abgeleiteten Ester oder ein solches Salz oder eine Hydroxygruppe tragen, wobei diese Reste mit in Anspruch 1 definierten Resten identisch sein oder zusätzliche Reste sein können, wobei die Reste, die jeweils mindestens eine Carbonsäuregruppe oder eine Hydroxygruppe tragen, bevorzugt zusätzlich entweder eine organisch polymerisierbare C=C-Doppelbindung oder eine Sulfonat- oder Sulfatgruppe tragen, nicht aber beide.

5. Kieselsäure(hetero)polykondensat nach einem der voranstehenden Ansprüche, worin die über ein Kohlenstoffatom an Silicium gebundenen Reste zumindest teilweise Alkylengruppen, Arylengruppen oder Alkylarylgruppe sind, die gegebenenfalls durch eine oder mehrere Gruppen -O-, -S-, -NH-, -S(O)-, -C(O)NH-, -NHC(O)-, -C(O)O-, -C(O)S, -NHC(O)NH- oder -C(O)NHC(O)- unterbrochen sein können.

6. Kieselsäure(hetero)polykondensat nach einem der voranstehenden Ansprüche, hergestellt unter der zusätzlichen Verwendung mindestens einer hydrolytisch kondensierbaren Metallverbindung eines Metalls, ausgewählt unter Metallen der III. und der IV. Hauptgruppe sowie der III. bis VI. Nebengruppe.

7. Kieselsäure(hetero)polykondensat nach einem der voranstehenden Ansprüche, **dadurch gekennzeichnet, dass** es wasserlöslich ist.

8. Komposit, umfassend eine Kieselsäure(hetero)polykondensat nach einem der voranstehenden Ansprüche sowie einen in das Kondensat eingearbeiteten Füllstoff.

9. Polymerisat, erhalten aus einem Kieselsäure(hetero)polykondensat nach einem der Ansprüche 1 bis 9 durch Polymerisation mindestens eines Teils von dessen (Meth)Acrylgruppen.

10. Verwendung eines Kieselsäure(hetero)polykondensats nach einem der Ansprüche 1 bis 7 oder eines Komposits nach Anspruch 8 oder eines Polymerisats nach Anspruch 9 für dentale Zwecke, insbesondere als Dentaladhäsiv.

11. Verfahren zum Herstellen eines Kieselsäure(hetero)polykondensats nach Anspruch 1, umfassend die Schritte
a. das Bereitstellen mindestens eines hydrolytisch kondensierbaren Silans mit einem über ein Kohlenstoffatom an Silicium gebundenen Rest, der eine organisch polymerisierbare C=C-Doppelbindung trägt, mit Ausnahme von Silanen, in denen die C=C-Doppelbindungen ausschließlich durch Methacrylester gebildet werden, die in Form einer Methylenacrylestergruppe an die über Kohlenstoff an Silicium gebundenen Gruppen angebunden sind, und mindestens eines hydrolytisch kondensierbaren Silans mit einem über ein Kohlenstoffatom an Silicium gebundenen Rest, der eine Sulfonat- oder Sulfatgruppe der Formel -(O)_{d}-SO₃M mit d = 0 oder 1 und M = Wasserstoff oder ein einwertiges Metallkation oder der entsprechende Anteil eines mehrwertigen Metallkations trägt, und
b. das Cokondensieren der genannten Silane unter Hydrolysebedingungen, vorzugsweise nach dem Sol-Gel-Verfahren.

12. Verfahren zum Herstellen eines Kieselsäure(hetero)polykondensats nach Anspruch 1, umfassend die Schritte
a. Erzeugen oder Bereitstellen eines Kieselsäurepolykondensats aus mindestens einem Silan mit einem über ein Kohlenstoffatom an Silicium gebundenen Rest, der eine organisch polymerisierbare C=C-Doppelbindung trägt, mit Ausnahme von Silanen, in denen die C=C-Doppelbindungen ausschließlich durch Methylenacrylester gebildet werden, und
b. Umsetzen nur eines Teils des Silans mit einer Verbindung, die eine Sulf(on)atgruppe trägt und an der C=C-Doppelbindung angreifen kann derart, dass ein Teil der die C=C-Doppelbindung enthaltenden Gruppe in eine sulf(on)athaltige Gruppe überführt wird.

13. Verfahren nach Anspruch 12, worin die organisch polymerisierbare C=C-Doppelbindung eine Vinylgruppe oder Teil einer Allyl-, Acryl-, Methacryl-, ggf. substituierten Bicyclo[2.2.1]heptengruppe, ggf. substituierten Bicyclo[2.2.2]octengruppe sowie einer ggf. substituierten Oxabicyclo[2.2.1]heptengruppe ist und die Verbindung, die eine Sulf(on)atgruppe trägt und an der C=C-Doppelbindung angreifen kann, ein Thioalkansulfonat oder ein Aminoalkansulfonat ist.

14. Verfahren zum Herstellen eines Kieselsäure(hetero)polykondensats nach Anspruch 1, umfassend die Schritte
a. Erzeugen oder Bereitstellen eines Kieselsäurepolykondensats aus mindestens zwei verschiedenen Silanen, von denen eines einen mit einem über ein Kohlenstoffatom an Silicium gebundenen Rest aufweist, der mindestens eine organisch polymerisierbare C=C-Doppelbindung trägt, mit Ausnahme von Silanen, in denen die C=C-Doppelbindungen ausschließlich durch Methylenacrylester gebildet werden, und von denen ein zweites einen über ein Kohlenstoffatom an Silicium gebundenen Rest aufweist, der eine reaktive Gruppe trägt, und
b. Umsetzen der reaktiven Gruppe des zweiten Silans mit einer Verbindung, die eine Sulf(on)atgruppe und einen Rest enthält, der an der reaktiven Gruppen des zweiten Silans unter Ausbildung einer Verknüpfung angreifen kann, derart, dass die Sulf(on)atgruppe in den über ein Kohlenstoffatom an Silicium gebundenen Rest eingeführt wird.

15. Verfahren nach Anspruch 14, worin die reaktive Gruppe des zweiten Silans ein gespannter Heteroring, insbesondere eine Epoxygruppe ist, und/oder worin die Verbindung, die eine Sulf(on)atgruppe und einen Rest enthält, der an der reaktiven Gruppen des zweiten Silans unter Ausbildung einer Verknüpfung angreifen kann, Natriumsulfat, Natriumsulfit oder ein primäres oder sekundäres Aminoalkansulf(on)at ist.

## Claims

1. A silicic acid (hetero) polycondensate from at least a first silane with a residue (1) bonded by a carbon atom to silicon and carrying an organically polymerizable C=C double bond, and from at least a second silane with a residue (2) bonded by a carbon atom to silicon and carrying a sulfonate group or sulfate group of the formula -(O)_{d}-SO₃M with d = 0 or 1 and with M = hydrogen or a monovalent metal cation or the corresponding portion of a multi-valent metal cation, wherein, when the organic polymerizable C=C double bond is contained in a (meth)acrylic group, this group is a (meth)acrylic ester, (meth)acrylic amide or (meth)acrylic thioester, with the exception of polycondensates in which the C=C double bonds are formed exclusively by methacrylic esters which are attached in the form of a methylene acrylic ester group to the groups that are bonded by a carbon to silicon.

2. The silicic acid (hetero) polycondensate according to claim 1, wherein the organically polymerizable C=C double bond is a vinyl group or part of an allyl group, acryl group, methacryl group, optionally substituted bicyclo[2.2.1] heptene group, optionally substituted bicyclo[2.2.2]octene group or an optionally substituted oxabicyclo[2.2.1]heptene group.

3. The silicic acid (hetero) polycondensate according to claim 1 or 2, comprising furthermore at least one silane with a residue bonded by a carbon atom to silicon and carrying an organically polymerizable C=C double bond as well as a sulfonate group or sulfate group.

4. The silicic acid (hetero) polycondensate according to one of the claims 1 to 3, comprising residues bonded by a carbon atom to silicon and carrying in each case at least one carboxylic acid group or an ester derived therefrom or a corresponding salt or a hydroxyl group, wherein these residues can be identical with the residues as defined in claim 1 or can be additional residues, wherein
the residues which carry in each case at least one carboxylic acid group or a hydroxyl group, carry in addition either an organically polymerizable C=C double bond or a sulfonate or sulfate group, but not both.

5. The silicic acid (hetero) polycondensate according to one of the preceding claims, wherein the residues bonded by a carbon atom to silicon are at least partially an alkylene group, arylene group or alkylaryl group that can be interrupted optionally by one or several groups -O-, -S-, -NH-, -S(O)-, -C(O)NH-, -NHC(O)-, -C(O)O-,-C(O)S, -NHC(O)NH- or -C(O)NHC(O)-.

6. The silicic acid (hetero) polycondensate according to one of the preceding claims, made with the additional use of at least one hydrolytically condensable metal compound of a metal, selected from metals of the main groups Ill and IV as well as from transition metal groups III to VI.

7. The silicic acid (hetero) polycondensate according to one of the preceding claims, **characterized in that** it is watersoluble.

8. A composite, comprising a silicic acid (hetero) polycondensate according to one of the preceding claims as well as a filler incorporated into the condensate.

9. A polymerisate obtained from a silicic acid (hetero) polycondensate according to one of the claims 1 to 8 by polymerization of at least some of its (meth)acryl groups.

10. The use of a silicic acid (hetero) polycondensate according to one of the claims 1 to 7 or a composite according to claim 8 or a polymerisate according to claim 10 for dental purposes, in particular as a dental adhesive.

11. A method for preparing a silicic acid (hetero) polycondensate according to claim 1, comprising the steps of
a. providing at least one hydrolytically condensable silane with a residue bonded by a carbon atom to silicon and carrying an organically polymerizable C=C double bond, with the exception of silanes in which the C=C double bonds are formed exclusively by methacrylic esters which are attached in the form of a methylene acrylic ester group to the groups that are bonded by a carbon to silicon, and providing at least one hydrolytically condensable silane with a residue bonded by a carbon atom to silicon and carrying a sulfonate group or sulfate group of the formula -(O)_{d}-SO₃M with d = 0 or 1 and with M = hydrogen or a monovalent metal cation or the corresponding portion of a multi-valent metal cation, and
b. co-condensing said silanes under hydrolytic conditions, preferably according to the sol gel process.

12. A method for preparing a silicic acid (hetero) polycondensate according to claim 1, comprising the steps
a. generating or providing a silicic acid polycondensate from at least one silane with a residue bonded by a carbon atom to silicon and carrying an organically polymerizable C=C double bond, with the exception of silanes in which the C=C double bonds are formed exclusively by methylene acrylic esters, and
b. reacting only a portion of the silane with a compound which carries a sulf(on)ate group and can attack at the C=C double bond so that some of the groups containing the C=C double bond are reacted to a sulf(on)ate-containing group.

13. The method according to claim 12, wherein the organically polymerizable C=C double bond is a vinyl group or part of an allyl group, acryl group, methacryl group, optionally substituted bicyclo[2.2.1]heptane group, optionally substituted bicyclo[2.2.2]octene group as well as an optionally substituted oxabicyclo[2.2.1]heptane group and the compound which carries a sulf(on)ate group and can attack the C=C double bond is a thioalkane sulfonate or an aminoalkane sulfonate.

14. A method for preparing a silicic acid (hetero) polycondensate according to claim 1, comprising the steps of
a. generating or providing a silicic acid polycondensate from at least two different silanes of which one has a residue bonded by a carbon atom to silicon and carrying at least one organically polymerizable C=C double bond, with the exception of silanes in which the C=C double bonds are formed exclusively by methylene acrylic esters, and of which a second one has a residue bonded by a carbon atom to silicon and carrying a reactive group, and
b. reacting the reactive group of the second silane with a compound which contains a sulf(on)ate group and a residue which can attack the reactive groups of the second silane with formation of a link so that the sulf(on)ate group is introduced into the residue that is bonded by a carbon atom to silicon.

15. The method according to claim 14, wherein the reactive group of the second silane is a strained hetero ring, in particular an epoxy group, and/or wherein the compound which contains a sulf(on)ate group and a residue that can attack the reactive groups of the second silanes with formation of a link, is sodium sulfate, sodium sulfite or a primary or secondary aminoalkane sulf(on)ate.

## Revendications

1. (Hétéro)polycondensat d'acide silicique comprenant au moins un premier silane présentant un radical (1) lié au silicium via un atome de carbone, qui porte une double liaison C=C organiquement polymérisable, et au moins un second silane présentant un radical (2) lié au silicium via un atome de carbone, ce radical portant un groupe sulfonate ou sulfate de formule -(O)_{d}-SO₃M dans laquelle d = 0 ou 1 et M est un hydrogène ou un cation métallique monovalent ou la fraction correspondante d'un cation métallique polyvalent, dans lequel le premier silane ne porte pas un radical (2) et le second silane ne porte pas un radical (1), dans lequel, dans le cas où la double liaison C=C organiquement polymérisable est contenue dans un groupe (méth)acrylique, ce groupe est présent sous forme d'ester, amide ou thioester de l'acide (méth)acrylique, à l'exception de polycondensats, dans lesquels les liaisons doubles C=C sont formées exclusivement par un ester méthacrylique, qui sont liés sous la forme d'un groupe ester méthylèneacrylique aux groupes liés au silicium via un carbone.

2. (Hétéro)polycondensat d'acide silicique selon la revendication 1, dans lequel la double liaison C=C organiquement polymérisable est un groupe vinyle ou une partie d'un groupe allyle, acryle, méthacryle, heptène éventuellement substitué bicyclo[2.2.1], octène éventuellement substitué bicyclo[2.2.2] ou un groupe heptène éventuellement substitué oxabicyclo[2.2.1].

3. (Hétéro)polycondensat d'acide silicique selon la revendication 1 ou 2, comprenant en outre au moins un silane présentant un radical lié au silicium via un atome de carbone, qui porte aussi bien une double liaison C=C organiquement polymérisable qu'un groupe sulfonate ou sulfate.

4. (Hétéro)polycondensat d'acide silicique selon l'une quelconque des revendications 1 à 3, comprenant des radicaux liés au silicium via un atome de carbone, qui portent respectivement au moins un groupe acide carboxylique ou un ester dérivé de celui-ci ou un tel sel ou un groupe hydroxy, dans lequel ces radicaux peuvent être identiques à des radicaux définis dans la revendication 1 ou être des radicaux additionnels, dans lequel les radicaux, qui portent respectivement au moins un groupe acide carboxylique ou un groupe hydroxy, portent de préférence en plus soit une double liaison C=C organiquement polymérisable soit un groupe sulfonate ou sulfate, mais pas les deux.

5. (Hétéro)polycondensat d'acide silicique selon l'une quelconque des revendications précédentes, dans lequel les radicaux liés au silicium via un atome de carbone sont au moins en partie des groupes alkylène, des groupes arylène ou un groupe alkylaryle, qui peuvent éventuellement être interrompus par un ou plusieurs groupe (s) -O-, -S-, -NH-, -S(O)-, -C(O)NH-, -NHC(O)-, -C(O)O-, -C(O)S-, -NHC(O)NH- ou -C(O)NHC(O)-.

6. (Hétéro)polycondensat d'acide silicique selon l'une quelconque des revendications précédentes, produit avec l'utilisation additionnelle d'au moins un composé métallique condensable par hydrolyse d'un métal, choisi parmi les métaux des groupes principaux III et IV ainsi que des sous-groupes III à VI.

7. (Hétéro)polycondensat d'acide silicique selon l'une quelconque des revendications précédentes, **caractérisé en ce qu'**il est hydrosoluble.

8. Composite, comprenant un (hétéro)polycondensat d'acide silicique selon l'une quelconque des revendications précédentes ainsi qu'une matière de charge incorporée dans le condensat.

9. Polymérisat, obtenu à partir d'un (hétéro)polycondensat d'acide silicique selon l'une quelconque des revendications 1 à 8 par polymérisation d'au moins une partie de ses groupes (méth)acryle.

10. Utilisation d'un (hétéro)polycondensat d'acide silicique selon l'une quelconque des revendications 1 à 7 ou d'un composite selon la revendication 8 ou d'un polymérisat selon la revendication 9 à des fins dentaires, en particulier comme adhésif dentaire.

11. Procédé de production d'un (hétéro)polycondensat d'acide silicique selon la revendication 1, comprenant les étapes suivantes:
a. préparation d'au moins un silane condensable par hydrolyse présentant un radical lié au silicium via un atome de carbone, qui porte une double liaison C=C organiquement polymérisable, à l'exception de silanes, dans lesquels les doubles liaisons C=C sont formées exclusivement par des esters méthacryliques, qui sont liés, sous forme d'un groupe ester méthylèneacrylique, aux groupes liés au silicium via un carbone, et d'au moins un silane condensable par hydrolyse présentant un radical lié au silicium via un atome de carbone, ce radical portant un groupe sulfonate ou sulfate de formule -(O)_{d}-SO₃M dans laquelle d = 0 ou 1 et M est un hydrogène ou un cation métallique monovalent ou la fraction correspondante d'un cation métallique polyvalent, et
b. co-condensation des silanes précités dans des conditions d'hydrolyse, de préférence selon le procédé sol-gel.

12. Procédé de production d'un (hétéro)polycondensat d'acide silicique selon la revendication 1, comprenant les étapes suivantes:
a. production ou préparation d'un polycondensat d'acide silicique à partir d'au moins un silane présentant un radical lié au silicium via un atome de carbone, qui porte une double liaison C=C organiquement polymérisable, à l'exception de silanes, dans lesquels les doubles liaisons C=C sont formées exclusivement par des esters méthylèneacryliques, et
b. conversion uniquement d'une partie du silane avec un composé, qui porte un groupe sulf(on)ate et qui peut réagir avec la double liaison C=C, de telle manière qu'une partie du groupe contenant la double liaison C=C soit transférée dans un groupe contenant le sulf(on)ate.

13. Procédé selon la revendication 12, dans lequel la double liaison C=C organiquement polymérisable est un groupe vinyle ou une partie d'un groupe allyle, acryle, méthacryle, heptène éventuellement substitué bicyclo[2.2.1], octène éventuellement substitué bicyclo[2.2.2] ou un groupe heptène éventuellement substitué oxabicyclo[2.2.1] et le composé, qui porte un groupe sulf(on)ate et qui peut réagir avec la double liaison C=C, est un sulfonate de thioalcane ou un sulfonate d'aminoalcane.

14. Procédé de production d'un (hétéro)polycondensat d'acide silicique selon la revendication 1, comprenant les étapes suivantes:
a. production ou préparation d'un polycondensat d'acide silicique à partir d'au moins deux silanes différents, dont l'un présente un radical lié au silicium via un atome de carbone, qui porte une double liaison C=C organiquement polymérisable, à l'exception de silanes, dans lesquels les doubles liaisons C=C sont formées exclusivement par des esters méthylèneacryliques, et dont un second présente un radical lié au silicium via un atome de carbone, qui porte un groupe réactif, et
b. conversion du groupe réactif du second silane avec un composé, qui porte un groupe sulf(on)ate et un radical, qui peut réagir avec le groupe réactif du second silane en formant une liaison, de telle manière que le groupe sulf(on)ate soit introduit dans le radical lié au silicium via un atome de carbone.

15. Procédé selon la revendication 14, dans lequel le groupe réactif du second silane est un hétérocycle tendu, en particulier un groupe époxy, et/ou dans lequel le composé, qui contient un groupe sulf(on)ate et un radical, qui peut réagir avec le groupe réactif du second silane en formant une liaison, est le sulfate de sodium, le sulfite de sodium ou un sulf(on)ate primaire ou secondaire d'aminoalcane.
